# EUROPEAN PATENT SPECIFICATION

(11) **EP 2 738 264 B1**
(45) Date of publication and mention of the grant of the patent: **20.01.2016**
(21) Application number: 13194017.3
(22) Date of filing: 22.11.2013
(51) Int. Cl.: C12Q 1/68

(54) **A method and system for determining behavior of thyroid tumor**
Verfahren und System zur Bestimmung des Verhaltens eines Schilddrüsentumors
Procédé et système permettant de déterminer le comportement d'une tumeur thyroïdienne

(30) Priority: 29.11.2012 JP 2012261300; 05.11.2013 JP 2013229365
(43) Date of publication of application: 04.06.2014
(73) Proprietor: SYSMEX CORPORATION, Kobe-shi, Hyogo 651-0073 (JP)
(72) Inventor: Shimizu, Hiroyuki, Hyogo, 651-0073 (JP); Nakabayashi, Kazuki, Hyogo, 651-0073 (JP); Daito, Motonari, Hyogo, 651-0073 (JP)
(74) Representative: Hoffmann Eitle

(56) References cited:
- EP-A1- 2 366 800
- WO-A2-2010/030365
- WO-A2-2010/056374
- FINLEY DAVID J ET AL: "Advancing the molecular diagnosis of thyroid nodules: defining benign lesions by molecular profiling", THYROID, MARY ANN LIEBERT, NEW YORK, NY, US, vol. 15, no. 6, 1 June 2005 (2005-06-01), pages 562-568, XP009087256, ISSN: 1050-7256, DOI: 10.1089/THY.2005.15.562
- JARZAB B ET AL: "Gene expression profile of papillary thyroid cancer: sources of variability and diagnostic implications", CANCER RESEARCH, AMERICAN ASSOCIATION FOR CANCER RESEARCH, US, vol. 65, no. 4, 15 February 2005 (2005-02-15), pages 1587-1597, XP002465072, ISSN: 0008-5472, DOI: 10.1158/0008-5472.CAN-04-3078

### SEQUENCE LISTING

<110> SYSMEX CORPORATION
<120> Method for determining the property of thyroid tumor and marker set fot the method
<130> 12-031JP1
<150> JP 2012-261300
   <151> 2012-11-29
<160> 419
<170> PatentIn version 3.5
<210> 1
   <211> 25
   <212> DNA
   <213> Homo sapiens
<400> 1
   gactacccat catattcagt gtccc 25
<210> 2
   <211> 25
   <212> DNA
   <213> Homo sapiens
<400> 2
   gtccctgttc ctcactcaga gagga 25
<210> 3
   <211> 25
   <212> DNA
   <213> Homo sapiens
<400> 3
   gtcccttcat ttcttgagac tcaac 25
<210> 4
   <211> 25
   <212> DNA
   <213> Homo sapiens
<400> 4
   ggtctcaaga gttgtccctg gaagg 25
<210> 5
   <211> 25
   <212> DNA
   <213> Homo sapiens
<400> 5
   gggcagtctg catctatttc aggtt 25
<210> 6
   <211> 25
   <212> DNA
   <213> Homo sapiens
<400> 6
   tatttcaggt tgtggctctt ggttc 25
<210> 7
   <211> 25
   <212> DNA
   <213> Homo sapiens
<400> 7
   ctcttggttc taggactctt acttc 25
<210> 8
   <211> 25
   <212> DNA
   <213> Homo sapiens
<400> 8
   tctggctaag ggctcagctt cttgg 25
<210> 9
   <211> 25
   <212> DNA
   <213> Homo sapiens
<400> 9
   agcttcttgg gacttcaacc atctt 25
<210> 10
   <211> 25
   <212> DNA
   <213> Homo sapiens
<400> 10
   agtatggctt tgtccctatg actca 25
<210> 11
   <211> 25
   <212> DNA
   <213> Homo sapiens
<400> 11
   ctgggatatc atctctacag cctgc 25
<210> 12
   <211> 25
   <212> DNA
   <213> Homo sapiens
<400> 12
   gaacacataa tccccgtggt ggtca 25
<210> 13
   <211> 25
   <212> DNA
   <213> Homo sapiens
<400> 13
   cagctcctgg ttcgagtgat cgtgt 25
<210> 14
   <211> 25
   <212> DNA
   <213> Homo sapiens
<400> 14
   tgatcgtgtg tcgctgcaac gtgga 25
<210> 15
   <211> 25
   <212> DNA
   <213> Homo sapiens
<400> 15
   gcagtgcatg cgcaaggtgg gccgc 25
<210> 16
   <211> 25
   <212> DNA
   <213> Homo sapiens
<400> 16
   catgcccacg aagctgtcgg cagtg 25
<210> 17
   <211> 25
   <212> DNA
   <213> Homo sapiens
<400> 17
   ttttcaccca ctggaccatg tcaag 25
<210> 18
   <211> 25
   <212> DNA
   <213> Homo sapiens
<400> 18
   gaagaaggac ccggatcaac cagca 25
<210> 19
   <211> 25
   <212> DNA
   <213> Homo sapiens
<400> 19
   gaaggcgact gtctgaatgg cccag 25
<210> 20
   <211> 25
   <212> DNA
   <213> Homo sapiens
<400> 20
   ggcccaggca gctctagctg ggagc 25
<210> 21
   <211> 25
   <212> DNA
   <213> Homo sapiens
<400> 21
   ctccatctga gtcccctggg agaga 25
<210> 22
   <211> 25
   <212> DNA
   <213> Homo sapiens
<400> 22
   ggacaccaac tttatggact gccca 25
<210> 23
   <211> 25
   <212> DNA
   <213> Homo sapiens
<400> 23
   gattggactc acaattatcg cctgc 25
<210> 24
   <211> 25
   <212> DNA
   <213> Homo sapiens
<400> 24
   ttatcgcctg ctttgctgtg atagt 25
<210> 25
   <211> 25
   <212> DNA
   <213> Homo sapiens
<400> 25
   agtggcgtga agaagctgca ttggc 25
<210> 26
   <211> 25
   <212> DNA
   <213> Homo sapiens
<400> 26
   gaagctgcat tggctgcaca ggcta 25
<210> 27
   <211> 25
   <212> DNA
   <213> Homo sapiens
<400> 27
   ctgaatacca tccctgtcat cagca 25
<210> 28
   <211> 25
   <212> DNA
   <213> Homo sapiens
<400> 28
   taccaaaata tctgccatgg tttta 25
<210> 29
   <211> 25
   <212> DNA
   <213> Homo sapiens
<400> 29
   tttatgtcgt gaacacctgt gtaac 25
<210> 30
   <211> 25
   <212> DNA
   <213> Homo sapiens
<400> 30
   ccattacctc ccaaacatag cagtt 25
<210> 31
   <211> 25
   <212> DNA
   <213> Homo sapiens
<400> 31
   gcagtttttc tgagtttcat cacct 25
<210> 32
   <211> 25
   <212> DNA
   <213> Homo sapiens
<400> 32
   gagtttcatc acctttgatt cattt 25
<210> 33
   <211> 25
   <212> DNA
   <213> Homo sapiens
<400> 33
   tttgattcat tttgcctgtt tttga 25
<210> 34
   <211> 25
   <212> DNA
   <213> Homo sapiens
<400> 34
   caacgtctaa ctggacttcc caaga 25
<210> 35
   <211> 25
   <212> DNA
   <213> Homo sapiens
<400> 35
   gataaatggt accagcgtcc tctta 25
<210> 36
   <211> 25
   <212> DNA
   <213> Homo sapiens
<400> 36
   aagatgcctt aatccattcc ttgag 25
<210> 37
   <211> 25
   <212> DNA
   <213> Homo sapiens
<400> 37
   ccttctgctt ctgagttgca catta 25
<210> 38
   <211> 25
   <212> DNA
   <213> Homo sapiens
<400> 38
   gcttccagac tctttgcgtt ggaga 25
<210> 39
   <211> 25
   <212> DNA
   <213> Homo sapiens
<400> 39
   gttaggatct tcaagtccca tcata 25
<210> 40
   <211> 25
   <212> DNA
   <213> Homo sapiens
<400> 40
   gggattgctt tcatctaatt ctggc 25
<210> 41
   <211> 25
   <212> DNA
   <213> Homo sapiens
<400> 41
   aggacctcac caaaagatcc agcct 25
<210> 42
   <211> 25
   <212> DNA
   <213> Homo sapiens
<400> 42
   agcctcatac ctacatcaga caaaa 25
<210> 43
   <211> 25
   <212> DNA
   <213> Homo sapiens
<400> 43
   caaaatatcg ccgttgttcc ttctg 25
<210> 44
   <211> 25
   <212> DNA
   <213> Homo sapiens
<400> 44
   tttgctttgg aaacacccac tcact 25
<210> 45
   <211> 25
   <212> DNA
   <213> Homo sapiens
<400> 45
   gatacttgct ccattcatac acaac 25
<210> 46
   <211> 25
   <212> DNA
   <213> Homo sapiens
<400> 46
   gcagtcactt tgacaggttc ctttt 25
<210> 47
   <211> 25
   <212> DNA
   <213> Homo sapiens
<400> 47
   ggttcctttt aagtggactc tctga 25
<210> 48
   <211> 25
   <212> DNA
   <213> Homo sapiens
<400> 48
   ggcttggatg tttttagttc tgact 25
<210> 49
   <211> 25
   <212> DNA
   <213> Homo sapiens
<400> 49
   ataatgactg gtcttcctta cctgg 25
<210> 50
   <211> 25
   <212> DNA
   <213> Homo sapiens
<400> 50
   gttagtttta gaattacacc acaag 25
<210> 51
   <211> 25
   <212> DNA
   <213> Homo sapiens
<400> 51
   atattgtttt gcattgtctg ttggc 25
<210> 52
   <211> 25
   <212> DNA
   <213> Homo sapiens
<400> 52
   gtgaactgtc atgatacgct taagg 25
<210> 53
   <211> 25
   <212> DNA
   <213> Homo sapiens
<400> 53
   atatattttt actgctttct gaatg 25
<210> 54
   <211> 25
   <212> DNA
   <213> Homo sapiens
<400> 54
   gcttttttaa ctcatccgtt tgcca 25
<210> 55
   <211> 25
   <212> DNA
   <213> Homo sapiens
<400> 55
   actcatccgt ttgccaatca ttgca 25
<210> 56
   <211> 25
   <212> DNA
   <213> Homo sapiens
<400> 56
   atctatgcta aaatgagccc tttcc 25
<210> 57
   <211> 25
   <212> DNA
   <213> Homo sapiens
<400> 57
   aagtcaccct atcaggtttc aaaca 25
<210> 58
   <211> 25
   <212> DNA
   <213> Homo sapiens
<400> 58
   gaaatttccc ttattcaggt ttctg 25
<210> 59
   <211> 25
   <212> DNA
   <213> Homo sapiens
<400> 59
   aatgaaagat taaccttccc cactg 25
<210> 60
   <211> 25
   <212> DNA
   <213> Homo sapiens
<400> 60
   actggtgatg acctaggcag gaatc 25
<210> 61
   <211> 25
   <212> DNA
   <213> Homo sapiens
<400> 61
   ggcaagcagg tgtgaagagc agggc 25
<210> 62
   <211> 25
   <212> DNA
   <213> Homo sapiens
<400> 62
   agcagggctc agcagcaagt cacat 25
<210> 63
   <211> 25
   <212> DNA
   <213> Homo sapiens
<400> 63
   catttttcta ctatttgacc aaaag 25
<210> 64
   <211> 25
   <212> DNA
   <213> Homo sapiens
<400> 64
   agaactctgg agtggtctaa gactg 25
<210> 65
   <211> 25
   <212> DNA
   <213> Homo sapiens
<400> 65
   atcagaccaa cagattttcc caacc 25
<210> 66
   <211> 25
   <212> DNA
   <213> Homo sapiens
<400> 66
   gattttccca acccaagact attgt 25
<210> 67
   <211> 25
   <212> DNA
   <213> Homo sapiens
<400> 67
   ggaagcttca tctgaccaat gtggg 25
<210> 68
   <211> 25
   <212> DNA
   <213> Homo sapiens
<400> 68
   cttgtgaaat gtgtccctaa gcctc 25
<210> 69
   <211> 25
   <212> DNA
   <213> Homo sapiens
<400> 69
   ccacccaccc aggtgtctaa gatag 25
<210> 70
   <211> 25
   <212> DNA
   <213> Homo sapiens
<400> 70
   agataggaca tgctcctttc tttct 25
<210> 71
   <211> 25
   <212> DNA
   <213> Homo sapiens
<400> 71
   tctctaatcc catcctgagg ttgcc 25
<210> 72
   <211> 25
   <212> DNA
   <213> Homo sapiens
<400> 72
   gcaaagccaa tatgaccact actga 25
<210> 73
   <211> 25
   <212> DNA
   <213> Homo sapiens
<400> 73
   aaatgcaagg gtcttaccct cctct 25
<210> 74
   <211> 25
   <212> DNA
   <213> Homo sapiens
<400> 74
   ttgccatccc ccaaatgtgt ggtat 25
<210> 75
   <211> 25
   <212> DNA
   <213> Homo sapiens
<400> 75
   tggtatggtg aaactaatcc cctga 25
<210> 76
   <211> 25
   <212> DNA
   <213> Homo sapiens
<400> 76
   atcccctgaa tgtgaattgc tatcc 25
<210> 77
   <211> 25
   <212> DNA
   <213> Homo sapiens
<400> 77
   ttgctatcct tattgcccta ttaaa 25
<210> 78
   <211> 25
   <212> DNA
   <213> Homo sapiens
<400> 78
   aagctttttg ctgtccatga atcct 25
<210> 79
   <211> 25
   <212> DNA
   <213> Homo sapiens
<400> 79
   catgaatcct ctgtggcaac tgtaa 25
<210> 80
   <211> 25
   <212> DNA
   <213> Homo sapiens
<400> 80
   gtcatgtctc ttgtgccatg gagct 25
<210> 81
   <211> 25
   <212> DNA
   <213> Homo sapiens
<400> 81
   gctactgagt ggtgctttat ctgaa 25
<210> 82
   <211> 25
   <212> DNA
   <213> Homo sapiens
<400> 82
   atgggactgg ctttccacag gaagt 25
<210> 83
   <211> 25
   <212> DNA
   <213> Homo sapiens
<400> 83
   gtaaactgct tcagagccca cagtc 25
<210> 84
   <211> 25
   <212> DNA
   <213> Homo sapiens
<400> 84
   ccctgctcag tgtccggaaa gaagt 25
<210> 85
   <211> 25
   <212> DNA
   <213> Homo sapiens
<400> 85
   ggaaagaagt cagtcatccc tgttg 25
<210> 86
   <211> 25
   <212> DNA
   <213> Homo sapiens
<400> 86
   ccctgttggc agtaaatctt cccac 25
<210> 87
   <211> 25
   <212> DNA
   <213> Homo sapiens
<400> 87
   tcccacaggc cgtccattag agatt 25
<210> 88
   <211> 25
   <212> DNA
   <213> Homo sapiens
<400> 88
   gtgctatttc atatctgtac tccag 25
<210> 89
   <211> 25
   <212> DNA
   <213> Homo sapiens
<400> 89
   agcagatctc atgctctcag atagg 25
<210> 90
   <211> 25
   <212> DNA
   <213> Homo sapiens
<400> 90
   gttcttttgt cacagtgctg gctct 25
<210> 91
   <211> 25
   <212> DNA
   <213> Homo sapiens
<400> 91
   cggggcttgc ttggttaacc acaga 25
<210> 92
   <211> 25
   <212> DNA
   <213> Homo sapiens
<400> 92
   ggaagcacgg ttactgtgtt ctcta 25
<210> 93
   <211> 25
   <212> DNA
   <213> Homo sapiens
<400> 93
   taaagatgta acctccacac ctttc 25
<210> 94
   <211> 25
   <212> DNA
   <213> Homo sapiens
<400> 94
   tccacacctt tctccagatt gggtg 25
<210> 95
   <211> 25
   <212> DNA
   <213> Homo sapiens
<400> 95
   aaggtggtgg gagtatctgt cgggg 25
<210> 96
   <211> 25
   <212> DNA
   <213> Homo sapiens
<400> 96
   ggcccttgga tgggtcaggt gggtg 25
<210> 97
   <211> 25
   <212> DNA
   <213> Homo sapiens
<400> 97
   tgagctcaaa gttgtcctac tgcca 25
<210> 98
   <211> 25
   <212> DNA
   <213> Homo sapiens
<400> 98
   tattttgcac ttgttactgt accat 25
<210> 99
   <211> 25
   <212> DNA
   <213> Homo sapiens
<400> 99
   tctgtatgtg ggatctgtgc ttggg 25
<210> 100
   <211> 25
   <212> DNA
   <213> Homo sapiens
<400> 100
   tgctgaggag tacgtgggcc tgtct 25
<210> 101
   <211> 25
   <212> DNA
   <213> Homo sapiens
<400> 101
   caaggacagg gtggactgcg gctac 25
<210> 102
   <211> 25
   <212> DNA
   <213> Homo sapiens
<400> 102
   cccaaggagt gcaacaaccg gggct 25
<210> 103
   <211> 25
   <212> DNA
   <213> Homo sapiens
<400> 103
   tccaggatcc ctggagtgcc ttggt 25
<210> 104
   <211> 25
   <212> DNA
   <213> Homo sapiens
<400> 104
   tggagtgcct tggtgtttca agccc 25
<210> 105
   <211> 25
   <212> DNA
   <213> Homo sapiens
<400> 105
   gtttcaagcc cctgactagg aagac 25
<210> 106
   <211> 25
   <212> DNA
   <213> Homo sapiens
<400> 106
   cagaatgcac cttctgaggc acctc 25
<210> 107
   <211> 25
   <212> DNA
   <213> Homo sapiens
<400> 107
   ctgtgattgc tgccaggcac tgttc 25
<210> 108
   <211> 25
   <212> DNA
   <213> Homo sapiens
<400> 108
   attgctgcca ggcactgttc atctc 25
<210> 109
   <211> 25
   <212> DNA
   <213> Homo sapiens
<400> 109
   tcccggcaag ctttctgctg aaagt 25
<210> 110
   <211> 25
   <212> DNA
   <213> Homo sapiens
<400> 110
   aagttcatat ctggagcctg atgtc 25
<210> 111
   <211> 25
   <212> DNA
   <213> Homo sapiens
<400> 111
   gagaaattac tcctcagatt cttgc 25
<210> 112
   <211> 25
   <212> DNA
   <213> Homo sapiens
<400> 112
   aatactctta agtgcatgta atcaa 25
<210> 113
   <211> 25
   <212> DNA
   <213> Homo sapiens
<400> 113
   aggggtgtca ctgttttcat cccct 25
<210> 114
   <211> 25
   <212> DNA
   <213> Homo sapiens
<400> 114
   ggaccccaac ttcagagggt gcagc 25
<210> 115
   <211> 25
   <212> DNA
   <213> Homo sapiens
<400> 115
   gggtgcagcc tctgaaagag actgt 25
<210> 116
   <211> 25
   <212> DNA
   <213> Homo sapiens
<400> 116
   gagactgtga atctatatat accat 25
<210> 117
   <211> 25
   <212> DNA
   <213> Homo sapiens
<400> 117
   ttctgggacg aattcatctt ctgag 25
<210> 118
   <211> 25
   <212> DNA
   <213> Homo sapiens
<400> 118
   ttccatctga attgcctcct agata 25
<210> 119
   <211> 25
   <212> DNA
   <213> Homo sapiens
<400> 119
   aaatgctgca gctacattct tgcct 25
<210> 120
   <211> 25
   <212> DNA
   <213> Homo sapiens
<400> 120
   ggactctagt tcagttttat atgca 25
<210> 121
   <211> 25
   <212> DNA
   <213> Homo sapiens
<400> 121
   atatgcaatg gatcactatt ttatt 25
<210> 122
   <211> 25
   <212> DNA
   <213> Homo sapiens
<400> 122
   ccatgaggct tgtccagacg cagtg 25
<210> 123
   <211> 25
   <212> DNA
   <213> Homo sapiens
<400> 123
   aggcttgtcc agacgcagtg aacca 25
<210> 124
   <211> 25
   <212> DNA
   <213> Homo sapiens
<400> 124
   gtccagacgc agtgaaccag tcgca 25
<210> 125
   <211> 25
   <212> DNA
   <213> Homo sapiens
<400> 125
   acgcagtgaa ccagtcgcag ctgat 25
<210> 126
   <211> 25
   <212> DNA
   <213> Homo sapiens
<400> 126
   gaaccagtcg cagctgataa cacac 25
<210> 127
   <211> 25
   <212> DNA
   <213> Homo sapiens
<400> 127
   gtcgcagctg ataacacaca gacca 25
<210> 128
   <211> 25
   <212> DNA
   <213> Homo sapiens
<400> 128
   ctgataacac acagaccatt cccga 25
<210> 129
   <211> 25
   <212> DNA
   <213> Homo sapiens
<400> 129
   tcccgatccc agaggtgcat ttcag 25
<210> 130
   <211> 25
   <212> DNA
   <213> Homo sapiens
<400> 130
   atcccagagg tgcatttcag gattc 25
<210> 131
   <211> 25
   <212> DNA
   <213> Homo sapiens
<400> 131
   ttctatttca tcagagacgg ggtct 25
<210> 132
   <211> 25
   <212> DNA
   <213> Homo sapiens
<400> 132
   ggcctcccag agggttggaa ttgca 25
<210> 133
   <211> 25
   <212> DNA
   <213> Homo sapiens
<400> 133
   gaattcgaca caatggggtt gcgtc 25
<210> 134
   <211> 25
   <212> DNA
   <213> Homo sapiens
<400> 134
   ggggttgcgt cacctatagt tttca 25
<210> 135
   <211> 25
   <212> DNA
   <213> Homo sapiens
<400> 135
   gcaactcaag gtgctcagct tgata 25
<210> 136
   <211> 25
   <212> DNA
   <213> Homo sapiens
<400> 136
   ggcaggggag aaactgactc ctttt 25
<210> 137
   <211> 25
   <212> DNA
   <213> Homo sapiens
<400> 137
   tctctttttt gcctgcacac ttaat 25
<210> 138
   <211> 25
   <212> DNA
   <213> Homo sapiens
<400> 138
   cacacttaat gtggactgcc tcctt 25
<210> 139
   <211> 25
   <212> DNA
   <213> Homo sapiens
<400> 139
   tggactgcct ccttggactt aaaaa 25
<210> 140
   <211> 25
   <212> DNA
   <213> Homo sapiens
<400> 140
   atttcttagc tttggacttg acaac 25
<210> 141
   <211> 25
   <212> DNA
   <213> Homo sapiens
<400> 141
   gacaactgac gaatgctctg ggggt 25
<210> 142
   <211> 25
   <212> DNA
   <213> Homo sapiens
<400> 142
   gtgtttatgt tgtcacttcg cttgc 25
<210> 143
   <211> 25
   <212> DNA
   <213> Homo sapiens
<400> 143
   cacctcaccc gatcttggtt ataag 25
<210> 144
   <211> 25
   <212> DNA
   <213> Homo sapiens
<400> 144
   ggatctgctc gtcatcattt ttcct 25
<210> 145
   <211> 25
   <212> DNA
   <213> Homo sapiens
<400> 145
   tcacgaatat tatgcccagt ttctg 25
<210> 146
   <211> 25
   <212> DNA
   <213> Homo sapiens
<400> 146
   atattatgcc cagtttctgc ctctg 25
<210> 147
   <211> 25
   <212> DNA
   <213> Homo sapiens
<400> 147
   attatgccca gtttctgcct ctgag 25
<210> 148
   <211> 25
   <212> DNA
   <213> Homo sapiens
<400> 148
   ttatgcccag tttctgcctc tgagg 25
<210> 149
   <211> 25
   <212> DNA
   <213> Homo sapiens
<400> 149
   tttctgcctc tgagggaaag cccag 25
<210> 150
   <211> 25
   <212> DNA
   <213> Homo sapiens
<400> 150
   cctctgaggg aaagcccaga aaagg 25
<210> 151
   <211> 25
   <212> DNA
   <213> Homo sapiens
<400> 151
   aggggcccag tcctggcctg gcttc 25
<210> 152
   <211> 25
   <212> DNA
   <213> Homo sapiens
<400> 152
   tggcctggct tctcctttgg aagtg 25
<210> 153
   <211> 25
   <212> DNA
   <213> Homo sapiens
<400> 153
   tggcttctcc tttggaagtg aggca 25
<210> 154
   <211> 25
   <212> DNA
   <213> Homo sapiens
<400> 154
   tcctttggaa gtgaggcatt gcacg 25
<210> 155
   <211> 25
   <212> DNA
   <213> Homo sapiens
<400> 155
   cctttggaag tgaggcattg cacgg 25
<210> 156
   <211> 25
   <212> DNA
   <213> Homo sapiens
<400> 156
   cattgcacgg ggagacgtac gtatc 25
<210> 157
   <211> 25
   <212> DNA
   <213> Homo sapiens
<400> 157
   ttgcacgggg agacgtacgt atcag 25
<210> 158
   <211> 25
   <212> DNA
   <213> Homo sapiens
<400> 158
   gacgtacgta tcagcggccc cttga 25
<210> 159
   <211> 25
   <212> DNA
   <213> Homo sapiens
<400> 159
   atcagcggcc ccttgactct gggga 25
<210> 160
   <211> 25
   <212> DNA
   <213> Homo sapiens
<400> 160
   ggcatccact ctgcggagga atgac 25
<210> 161
   <211> 25
   <212> DNA
   <213> Homo sapiens
<400> 161
   gaggaatgac tccatctacg aggcc 25
<210> 162
   <211> 25
   <212> DNA
   <213> Homo sapiens
<400> 162
   gaggagtata actacggctt cgtgg 25
<210> 163
   <211> 25
   <212> DNA
   <213> Homo sapiens
<400> 163
   gtataactac ggcttcgtgg tggag 25
<210> 164
   <211> 25
   <212> DNA
   <213> Homo sapiens
<400> 164
   aactacggct tcgtggtgga gaaga 25
<210> 165
   <211> 25
   <212> DNA
   <213> Homo sapiens
<400> 165
   cttcgccctg caaggcgttt gctat 25
<210> 166
   <211> 25
   <212> DNA
   <213> Homo sapiens
<400> 166
   cgccctgcaa ggcgtttgct atctt 25
<210> 167
   <211> 25
   <212> DNA
   <213> Homo sapiens
<400> 167
   gcaaggcgtt tgctatcttc agcca 25
<210> 168
   <211> 25
   <212> DNA
   <213> Homo sapiens
<400> 168
   ggagctggac agaagccagt gcctt 25
<210> 169
   <211> 25
   <212> DNA
   <213> Homo sapiens
<400> 169
   tggacagaag ccagtgcctt taagt 25
<210> 170
   <211> 25
   <212> DNA
   <213> Homo sapiens
<400> 170
   ctcctggcaa taaacactac ccggt 25
<210> 171
   <211> 25
   <212> DNA
   <213> Homo sapiens
<400> 171
   cggttcttca gttttctgag aagag 25
<210> 172
   <211> 25
   <212> DNA
   <213> Homo sapiens
<400> 172
   tcgcctctgc aggaaagagc agagc 25
<210> 173
   <211> 25
   <212> DNA
   <213> Homo sapiens
<400> 173
   cctctgcagg aaagagcaga gcccg 25
<210> 174
   <211> 25
   <212> DNA
   <213> Homo sapiens
<400> 174
   gcccagcacg catggtattg ccagc 25
<210> 175
   <211> 25
   <212> DNA
   <213> Homo sapiens
<400> 175
   tgtagtctct gctccctgca tttca 25
<210> 176
   <211> 25
   <212> DNA
   <213> Homo sapiens
<400> 176
   gactggcaac agaacagccg tggct 25
<210> 177
   <211> 25
   <212> DNA
   <213> Homo sapiens
<400> 177
   aacagccgtg gctgctttat ctctc 25
<210> 178
   <211> 25
   <212> DNA
   <213> Homo sapiens
<400> 178
   gggagcctgg ccacctcgca gctgt 25
<210> 179
   <211> 25
   <212> DNA
   <213> Homo sapiens
<400> 179
   ggagcctggc cacctcgcag ctgtt 25
<210> 180
   <211> 25
   <212> DNA
   <213> Homo sapiens
<400> 180
   ccctcaacct ttgaactgga actgc 25
<210> 181
   <211> 25
   <212> DNA
   <213> Homo sapiens
<400> 181
   aacctttgaa ctggaactgc tggct 25
<210> 182
   <211> 25
   <212> DNA
   <213> Homo sapiens
<400> 182
   agggttttcg acaactgcag ctgaa 25
<210> 183
   <211> 25
   <212> DNA
   <213> Homo sapiens
<400> 183
   ttttcgacaa ctgcagctga atctc 25
<210> 184
   <211> 25
   <212> DNA
   <213> Homo sapiens
<400> 184
   gacaactgca gctgaatctc atgga 25
<210> 185
   <211> 25
   <212> DNA
   <213> Homo sapiens
<400> 185
   tgaatctcat ggaaaagctg gattc 25
<210> 186
   <211> 25
   <212> DNA
   <213> Homo sapiens
<400> 186
   ggaaaagctg gattcctctg cctta 25
<210> 187
   <211> 25
   <212> DNA
   <213> Homo sapiens
<400> 187
   aggagggaag cctctgtgct gctca 25
<210> 188
   <211> 25
   <212> DNA
   <213> Homo sapiens
<400> 188
   gggaagcctc tgtgctgctc acctg 25
<210> 189
   <211> 25
   <212> DNA
   <213> Homo sapiens
<400> 189
   aaagcccagc tcagcgggta cgcag 25
<210> 190
   <211> 25
   <212> DNA
   <213> Homo sapiens
<400> 190
   cgcagtggag gcgacaagga accaa 25
<210> 191
   <211> 25
   <212> DNA
   <213> Homo sapiens
<400> 191
   ggaggcgaca aggaaccaat cacaa 25
<210> 192
   <211> 25
   <212> DNA
   <213> Homo sapiens
<400> 192
   gacaaggaac caatcacaac tacag 25
<210> 193
   <211> 25
   <212> DNA
   <213> Homo sapiens
<400> 193
   caatcacaac tacagaggtt tctct 25
<210> 194
   <211> 25
   <212> DNA
   <213> Homo sapiens
<400> 194
   tacagaggtt tctctgatcc tcctc 25
<210> 195
   <211> 25
   <212> DNA
   <213> Homo sapiens
<400> 195
   gaggtttctc tgatcctcct ccact 25
<210> 196
   <211> 25
   <212> DNA
   <213> Homo sapiens
<400> 196
   actcccgctg cttcaacttg actaa 25
<210> 197
   <211> 25
   <212> DNA
   <213> Homo sapiens
<400> 197
   gctgcttcaa cttgactaag cttaa 25
<210> 198
   <211> 25
   <212> DNA
   <213> Homo sapiens
<400> 198
   tgcgtcttcc caagttagca ggtgt 25
<210> 199
   <211> 25
   <212> DNA
   <213> Homo sapiens
<400> 199
   tcctggcaga agccatggcc catgt 25
<210> 200
   <211> 25
   <212> DNA
   <213> Homo sapiens
<400> 200
   agaaggccca gctcacatgt gagtc 25
<210> 201
   <211> 25
   <212> DNA
   <213> Homo sapiens
<400> 201
   aggcccagct cacatgtgag tcctg 25
<210> 202
   <211> 25
   <212> DNA
   <213> Homo sapiens
<400> 202
   cagaagccat ggcccatgtc tgcac 25
<210> 203
   <211> 25
   <212> DNA
   <213> Homo sapiens
<400> 203
   aaggcccagc tcacatgtga gtcct 25
<210> 204
   <211> 25
   <212> DNA
   <213> Homo sapiens
<400> 204
   gcagaagcca tggcccatgt ctgca 25
<210> 205
   <211> 25
   <212> DNA
   <213> Homo sapiens
<400> 205
   cccacgcccg aacagcaggg gcaga 25
<210> 206
   <211> 25
   <212> DNA
   <213> Homo sapiens
<400> 206
   tccttcgaag tgtgtccaag tccgc 25
<210> 207
   <211> 25
   <212> DNA
   <213> Homo sapiens
<400> 207
   tgtccaagtc cgcatttgag ccttg 25
<210> 208
   <211> 25
   <212> DNA
   <213> Homo sapiens
<400> 208
   ggctcactgt cctgagttgc agtaa 25
<210> 209
   <211> 25
   <212> DNA
   <213> Homo sapiens
<400> 209
   aggcccagcc ggaacgagat ttacg 25
<210> 210
   <211> 25
   <212> DNA
   <213> Homo sapiens
<400> 210
   agtgcgcatg cgtagatgga gcaaa 25
<210> 211
   <211> 25
   <212> DNA
   <213> Homo sapiens
<400> 211
   tagatggagc aaaaccgcct ctcca 25
<210> 212
   <211> 25
   <212> DNA
   <213> Homo sapiens
<400> 212
   atcgaccctt gggtccagca aggaa 25
<210> 213
   <211> 25
   <212> DNA
   <213> Homo sapiens
<400> 213
   cttgggtcca gcaaggaagc cctaa 25
<210> 214
   <211> 25
   <212> DNA
   <213> Homo sapiens
<400> 214
   ggaagcccta agtgacgtca acctt 25
<210> 215
   <211> 25
   <212> DNA
   <213> Homo sapiens
<400> 215
   gacgtcaacc tttgttccca tggta 25
<210> 216
   <211> 25
   <212> DNA
   <213> Homo sapiens
<400> 216
   ttcccatggt aacgcagaac cttgg 25
<210> 217
   <211> 25
   <212> DNA
   <213> Homo sapiens
<400> 217
   cggaaaaccg gctcagttga cgctg 25
<210> 218
   <211> 25
   <212> DNA
   <213> Homo sapiens
<400> 218
   cggctcagtt gacgctgcag gtaaa 25
<210> 219
   <211> 25
   <212> DNA
   <213> Homo sapiens
<400> 219
   gttgacgctg caggtaaaaa gactt 25
<210> 220
   <211> 25
   <212> DNA
   <213> Homo sapiens
<400> 220
   gtcacgggat ggatagccgg cctgc 25
<210> 221
   <211> 25
   <212> DNA
   <213> Homo sapiens
<400> 221
   atattgtgaa cgagccacct cctaa 25
<210> 222
   <211> 25
   <212> DNA
   <213> Homo sapiens
<400> 222
   catcaagaac ccagcggagc gggcg 25
<210> 223
   <211> 25
   <212> DNA
   <213> Homo sapiens
<400> 223
   gacctgaaga tgctcacaaa ccaca 25
<210> 224
   <211> 25
   <212> DNA
   <213> Homo sapiens
<400> 224
   acaaaccaca ccttcatcaa gcggt 25
<210> 225
   <211> 25
   <212> DNA
   <213> Homo sapiens
<400> 225
   ccttcatcaa gcggtccgag gtgga 25
<210> 226
   <211> 25
   <212> DNA
   <213> Homo sapiens
<400> 226
   gaagtggatt ttgccggctg gttgt 25
<210> 227
   <211> 25
   <212> DNA
   <213> Homo sapiens
<400> 227
   tgtgtaaaac cctgcggctg aacca 25
<210> 228
   <211> 25
   <212> DNA
   <213> Homo sapiens
<400> 228
   acagcgcatg caggaacggg ggtct 25
<210> 229
   <211> 25
   <212> DNA
   <213> Homo sapiens
<400> 229
   tgctgtgtgt ggtctcagag gctct 25
<210> 230
   <211> 25
   <212> DNA
   <213> Homo sapiens
<400> 230
   ctctgcttcc ttaggttaca aaaca 25
<210> 231
   <211> 25
   <212> DNA
   <213> Homo sapiens
<400> 231
   gtgacaacgt agagcgcgtg ctgaa 25
<210> 232
   <211> 25
   <212> DNA
   <213> Homo sapiens
<400> 232
   tgaaggtgct cacgggcacg ggtaa 25
<210> 233
   <211> 25
   <212> DNA
   <213> Homo sapiens
<400> 233
   tattcagcct aactatcctg cggca 25
<210> 234
   <211> 25
   <212> DNA
   <213> Homo sapiens
<400> 234
   ctttgccctg aacttgtccg ggtag 25
<210> 235
   <211> 25
   <212> DNA
   <213> Homo sapiens
<400> 235
   ggagggccgg aggcatgtgg cctcc 25
<210> 236
   <211> 25
   <212> DNA
   <213> Homo sapiens
<400> 236
   gacctcctga cctgggtggt tgagg 25
<210> 237
   <211> 25
   <212> DNA
   <213> Homo sapiens
<400> 237
   ggttgaggct caagacagct caccc 25
<210> 238
   <211> 25
   <212> DNA
   <213> Homo sapiens
<400> 238
   ggtccagaag ctccatgctg gtcac 25
<210> 239
   <211> 25
   <212> DNA
   <213> Homo sapiens
<400> 239
   atgctggtca ctagggtgct gtgct 25
<210> 240
   <211> 25
   <212> DNA
   <213> Homo sapiens
<400> 240
   ggcccaagca ggtgggagtg gacac 25
<210> 241
   <211> 25
   <212> DNA
   <213> Homo sapiens
<400> 241
   cctgctcagg cctcttgaga aatgg 25
<210> 242
   <211> 25
   <212> DNA
   <213> Homo sapiens
<400> 242
   gccgcaccct gagcatggac aaggg 25
<210> 243
   <211> 25
   <212> DNA
   <213> Homo sapiens
<400> 243
   ggacaagggc ttctgaggcc agcca 25
<210> 244
   <211> 25
   <212> DNA
   <213> Homo sapiens
<400> 244
   gcgccagggc tcagatgggg cctgg 25
<210> 245
   <211> 25
   <212> DNA
   <213> Homo sapiens
<400> 245
   tgggctgttg tgtctggagc ctgca 25
<210> 246
   <211> 25
   <212> DNA
   <213> Homo sapiens
<400> 246
   gagcctgcag gcaggtgggg gctgc 25
<210> 247
   <211> 25
   <212> DNA
   <213> Homo sapiens
<400> 247
   cctccggctg gtgtcttgag accag 25
<210> 248
   <211> 25
   <212> DNA
   <213> Homo sapiens
<400> 248
   ggacagagtg ccctggggca tccac 25
<210> 249
   <211> 25
   <212> DNA
   <213> Homo sapiens
<400> 249
   gctgggctca ctacatgggc cagcc 25
<210> 250
   <211> 25
   <212> DNA
   <213> Homo sapiens
<400> 250
   tgctggcgcc taggttgtgt tgaga 25
<210> 251
   <211> 25
   <212> DNA
   <213> Homo sapiens
<400> 251
   cacctgtcac cttttctaga aataa 25
<210> 252
   <211> 25
   <212> DNA
   <213> Homo sapiens
<400> 252
   accctgactg tggggtgcat cggtc 25
<210> 253
   <211> 25
   <212> DNA
   <213> Homo sapiens
<400> 253
   gaggcttctc cagagctggg agctg 25
<210> 254
   <211> 25
   <212> DNA
   <213> Homo sapiens
<400> 254
   tgtcacccag ccatggtgtg gtcca 25
<210> 255
   <211> 25
   <212> DNA
   <213> Homo sapiens
<400> 255
   tccaggcagg gacatctcgg taccc 25
<210> 256
   <211> 25
   <212> DNA
   <213> Homo sapiens
<400> 256
   tcgtccacaa acactccgtg gctga 25
<210> 257
   <211> 25
   <212> DNA
   <213> Homo sapiens
<400> 257
   ggctgagagg cagcggatcc aggca 25
<210> 258
   <211> 25
   <212> DNA
   <213> Homo sapiens
<400> 258
   atccaggcag cgatgctgag ccacc 25
<210> 259
   <211> 25
   <212> DNA
   <213> Homo sapiens
<400> 259
   accaccccgg aggacacgtg gatga 25
<210> 260
   <211> 25
   <212> DNA
   <213> Homo sapiens
<400> 260
   tgatggggtc agagatcact gagct 25
<210> 261
   <211> 25
   <212> DNA
   <213> Homo sapiens
<400> 261
   gcctccgtgg ctccaaggtg agggt 25
<210> 262
   <211> 25
   <212> DNA
   <213> Homo sapiens
<400> 262
   ggtgagggtc atcttcacga gcaaa 25
<210> 263
   <211> 25
   <212> DNA
   <213> Homo sapiens
<400> 263
   agtgacaacg aacgtctgag gcttc 25
<210> 264
   <211> 25
   <212> DNA
   <213> Homo sapiens
<400> 264
   ctccgaaggc ccagggaaca tacct 25
<210> 265
   <211> 25
   <212> DNA
   <213> Homo sapiens
<400> 265
   ccagcgccag gtgctcttgc ctttt 25
<210> 266
   <211> 25
   <212> DNA
   <213> Homo sapiens
<400> 266
   agcgccaggt gctcttgcct tttcc 25
<210> 267
   <211> 25
   <212> DNA
   <213> Homo sapiens
<400> 267
   gctcttgcct tttcccactg agaga 25
<210> 268
   <211> 25
   <212> DNA
   <213> Homo sapiens
<400> 268
   gccttttccc actgagagaa ggctg 25
<210> 269
   <211> 25
   <212> DNA
   <213> Homo sapiens
<400> 269
   ttcccactga gagaaggctg ctctt 25
<210> 270
   <211> 25
   <212> DNA
   <213> Homo sapiens
<400> 270
   tcccactgag agaaggctgc tcttt 25
<210> 271
   <211> 25
   <212> DNA
   <213> Homo sapiens
<400> 271
   ctgagagaag gctgctcttt tgtac 25
<210> 272
   <211> 25
   <212> DNA
   <213> Homo sapiens
<400> 272
   tgagagaagg ctgctctttt gtact 25
<210> 273
   <211> 25
   <212> DNA
   <213> Homo sapiens
<400> 273
   gagagaaggc tgctcttttg tactg 25
<210> 274
   <211> 25
   <212> DNA
   <213> Homo sapiens
<400> 274
   agagaaggct gctcttttgt actgc 25
<210> 275
   <211> 25
   <212> DNA
   <213> Homo sapiens
<400> 275
   gagaaggctg ctcttttgta ctgcc 25
<210> 276
   <211> 25
   <212> DNA
   <213> Homo sapiens
<400> 276
   agaaggctgc tcttttgtac tgccc 25
<210> 277
   <211> 25
   <212> DNA
   <213> Homo sapiens
<400> 277
   aggggtggcc agagccaaag ccagc 25
<210> 278
   <211> 25
   <212> DNA
   <213> Homo sapiens
<400> 278
   tggccagagc caaagccagc tactg 25
<210> 279
   <211> 25
   <212> DNA
   <213> Homo sapiens
<400> 279
   ccctcctgca ttcctcattt aagga 25
<210> 280
   <211> 25
   <212> DNA
   <213> Homo sapiens
<400> 280
   tattgagcac cctttgtgtg cagac 25
<210> 281
   <211> 25
   <212> DNA
   <213> Homo sapiens
<400> 281
   accctttgtg tgcagacatg gctcc 25
<210> 282
   <211> 25
   <212> DNA
   <213> Homo sapiens
<400> 282
   gacatggctc caggtgctta gcaat 25
<210> 283
   <211> 25
   <212> DNA
   <213> Homo sapiens
<400> 283
   gcttagcaat caatggtgag cgtgg 25
<210> 284
   <211> 25
   <212> DNA
   <213> Homo sapiens
<400> 284
   gtgagcgtgg tattccaggc taaag 25
<210> 285
   <211> 25
   <212> DNA
   <213> Homo sapiens
<400> 285
   attacaggct ggttgtggca gctca 25
<210> 286
   <211> 25
   <212> DNA
   <213> Homo sapiens
<400> 286
   tggttgtggc agctcatgac tgtaa 25
<210> 287
   <211> 25
   <212> DNA
   <213> Homo sapiens
<400> 287
   gtgctaacag ctctagctac tcagg 25
<210> 288
   <211> 25
   <212> DNA
   <213> Homo sapiens
<400> 288
   gcagcatcaa ccatctctga gataa 25
<210> 289
   <211> 25
   <212> DNA
   <213> Homo sapiens
<400> 289
   aacaacttgc ggctatccag atggc 25
<210> 290
   <211> 25
   <212> DNA
   <213> Homo sapiens
<400> 290
   atggccgggc aatggcaggg caggt 25
<210> 291
   <211> 25
   <212> DNA
   <213> Homo sapiens
<400> 291
   gcagggcagg tgccgtggcc ctgca 25
<210> 292
   <211> 25
   <212> DNA
   <213> Homo sapiens
<400> 292
   gagaatgatc tggtgcgaag cccca 25
<210> 293
   <211> 25
   <212> DNA
   <213> Homo sapiens
<400> 293
   tgcaccaggc tgttgaggac ctgcc 25
<210> 294
   <211> 25
   <212> DNA
   <213> Homo sapiens
<400> 294
   tgaggacctg ccagagggcg tggac 25
<210> 295
   <211> 25
   <212> DNA
   <213> Homo sapiens
<400> 295
   ccgcagggag ttctatctct cagac 25
<210> 296
   <211> 25
   <212> DNA
   <213> Homo sapiens
<400> 296
   cagactctga cttccaagat atctt 25
<210> 297
   <211> 25
   <212> DNA
   <213> Homo sapiens
<400> 297
   tctacagcat ggccacgtgg aggca 25
<210> 298
   <211> 25
   <212> DNA
   <213> Homo sapiens
<400> 298
   ggtcatcctc tgcgtgtcag taaaa 25
<210> 299
   <211> 25
   <212> DNA
   <213> Homo sapiens
<400> 299
   cagtcagagt ggacaaacct ctgtg 25
<210> 300
   <211> 25
   <212> DNA
   <213> Homo sapiens
<400> 300
   gacaaacctc tgtgggaggc tctac 25
<210> 301
   <211> 25
   <212> DNA
   <213> Homo sapiens
<400> 301
   tctgtgggag gctctactgc actga 25
<210> 302
   <211> 25
   <212> DNA
   <213> Homo sapiens
<400> 302
   ggaggctcta ctgcactgag atgca 25
<210> 303
   <211> 25
   <212> DNA
   <213> Homo sapiens
<400> 303
   tgcactgaga tgcagctctt ccgag 25
<210> 304
   <211> 25
   <212> DNA
   <213> Homo sapiens
<400> 304
   ggggctccta agccagtgta caact 25
<210> 305
   <211> 25
   <212> DNA
   <213> Homo sapiens
<400> 305
   agtgtacaac tgggtgcgtc ttgga 25
<210> 306
   <211> 25
   <212> DNA
   <213> Homo sapiens
<400> 306
   gtacaactgg gtgcgtcttg gaact 25
<210> 307
   <211> 25
   <212> DNA
   <213> Homo sapiens
<400> 307
   caactgggtg cgtcttggaa ctttt 25
<210> 308
   <211> 25
   <212> DNA
   <213> Homo sapiens
<400> 308
   gccaccaacc agatgggcag tgcat 25
<210> 309
   <211> 25
   <212> DNA
   <213> Homo sapiens
<400> 309
   aaccagatgg gcagtgcatc ctgtg 25
<210> 310
   <211> 25
   <212> DNA
   <213> Homo sapiens
<400> 310
   catgctgagc tcagacttca ggccc 25
<210> 311
   <211> 25
   <212> DNA
   <213> Homo sapiens
<400> 311
   ccacttcaac aagcacctgc tgggc 25
<210> 312
   <211> 25
   <212> DNA
   <213> Homo sapiens
<400> 312
   cagcagggct cggagcccag ctacc 25
<210> 313
   <211> 25
   <212> DNA
   <213> Homo sapiens
<400> 313
   ccgtcctgtg cagcaccatg gagaa 25
<210> 314
   <211> 25
   <212> DNA
   <213> Homo sapiens
<400> 314
   atggagaaga gcgtgctcgg cggcc 25
<210> 315
   <211> 25
   <212> DNA
   <213> Homo sapiens
<400> 315
   tgcgcgtccg tgtgacggag ctgga 25
<210> 316
   <211> 25
   <212> DNA
   <213> Homo sapiens
<400> 316
   gaggacgagg tgcgcaacct gcgca 25
<210> 317
   <211> 25
   <212> DNA
   <213> Homo sapiens
<400> 317
   acctgcgcaa gatcaatcgg gacct 25
<210> 318
   <211> 25
   <212> DNA
   <213> Homo sapiens
<400> 318
   atcaatcggg acctgttcga cttct 25
<210> 319
   <211> 25
   <212> DNA
   <213> Homo sapiens
<400> 319
   ggcacgcgtc acagtggtgc tagtc 25
<210> 320
   <211> 25
   <212> DNA
   <213> Homo sapiens
<400> 320
   agtggtgcta gtctgttttt aacaa 25
<210> 321
   <211> 25
   <212> DNA
   <213> Homo sapiens
<400> 321
   agaaatgcct cacagctatc gtgaa 25
<210> 322
   <211> 25
   <212> DNA
   <213> Homo sapiens
<400> 322
   gctatcgtga agtgcgccac aagca 25
<210> 323
   <211> 25
   <212> DNA
   <213> Homo sapiens
<400> 323
   gcgccacaag caaaccagct ttctt 25
<210> 324
   <211> 25
   <212> DNA
   <213> Homo sapiens
<400> 324
   aaaccagctt tctttgcaga gaagc 25
<210> 325
   <211> 25
   <212> DNA
   <213> Homo sapiens
<400> 325
   gcagagaagc ttcatcaagc catga 25
<210> 326
   <211> 25
   <212> DNA
   <213> Homo sapiens
<400> 326
   ggtgttggaa ctcgccataa ggcat 25
<210> 327
   <211> 25
   <212> DNA
   <213> Homo sapiens
<400> 327
   tatggtttcc cgttctgaaa ttgac 25
<210> 328
   <211> 25
   <212> DNA
   <213> Homo sapiens
<400> 328
   agaagatgta tggtatctcc ctttg 25
<210> 329
   <211> 25
   <212> DNA
   <213> Homo sapiens
<400> 329
   cctttgccaa gccatcctgg atgaa 25
<210> 330
   <211> 25
   <212> DNA
   <213> Homo sapiens
<400> 330
   gaaactaaac attcccttga tggtc 25
<210> 331
   <211> 25
   <212> DNA
   <213> Homo sapiens
<400> 331
   gatggtctca agctatgatc agaag 25
<210> 332
   <211> 25
   <212> DNA
   <213> Homo sapiens
<400> 332
   ctgtgccatc tttcactggg aggca 25
<210> 333
   <211> 25
   <212> DNA
   <213> Homo sapiens
<400> 333
   gtttttcatc ctgccatatg acacc 25
<210> 334
   <211> 25
   <212> DNA
   <213> Homo sapiens
<400> 334
   cacagtgagg agtagccagg ttgct 25
<210> 335
   <211> 25
   <212> DNA
   <213> Homo sapiens
<400> 335
   gttgctagga cagtaaccct gccac 25
<210> 336
   <211> 25
   <212> DNA
   <213> Homo sapiens
<400> 336
   tgccacacac tgcctgaaat cggaa 25
<210> 337
   <211> 25
   <212> DNA
   <213> Homo sapiens
<400> 337
   gcctgaaatc ggaactccct tggcc 25
<210> 338
   <211> 25
   <212> DNA
   <213> Homo sapiens
<400> 338
   cctccctctt aactaagtga cccat 25
<210> 339
   <211> 25
   <212> DNA
   <213> Homo sapiens
<400> 339
   gggaagggta ttggacacgg cagcg 25
<210> 340
   <211> 25
   <212> DNA
   <213> Homo sapiens
<400> 340
   gcagcgtcct ccttattgaa aacac 25
<210> 341
   <211> 25
   <212> DNA
   <213> Homo sapiens
<400> 341
   atacattttt catctcatgg ctaca 25
<210> 342
   <211> 25
   <212> DNA
   <213> Homo sapiens
<400> 342
   tactgatgat ccttccactt atatt 25
<210> 343
   <211> 25
   <212> DNA
   <213> Homo sapiens
<400> 343
   gaatgtattt gctgacaacc attaa 25
<210> 344
   <211> 25
   <212> DNA
   <213> Homo sapiens
<400> 344
   gaagttttac tccaggttat tgcaa 25
<210> 345
   <211> 25
   <212> DNA
   <213> Homo sapiens
<400> 345
   tcatgtccca gaacttagcc tttac 25
<210> 346
   <211> 25
   <212> DNA
   <213> Homo sapiens
<400> 346
   ttagccttta cctgtgaagt gttac 25
<210> 347
   <211> 25
   <212> DNA
   <213> Homo sapiens
<400> 347
   gaagtgttac tacagcctta atatt 25
<210> 348
   <211> 25
   <212> DNA
   <213> Homo sapiens
<400> 348
   tacagcctta atattttcct agtag 25
<210> 349
   <211> 25
   <212> DNA
   <213> Homo sapiens
<400> 349
   gtgtttttag ctgtgacaca actgt 25
<210> 350
   <211> 25
   <212> DNA
   <213> Homo sapiens
<400> 350
   taactcaagg ataccttctt attta 25
<210> 351
   <211> 25
   <212> DNA
   <213> Homo sapiens
<400> 351
   aacatttata tgtagccttt actgt 25
<210> 352
   <211> 25
   <212> DNA
   <213> Homo sapiens
<400> 352
   tagcctttac tgtttgatat accaa 25
<210> 353
   <211> 25
   <212> DNA
   <213> Homo sapiens
<400> 353
   ctcattactt atactgggac accat 25
<210> 354
   <211> 25
   <212> DNA
   <213> Homo sapiens
<400> 354
   acagctttcc aaggtgacaa actcc 25
<210> 355
   <211> 25
   <212> DNA
   <213> Homo sapiens
<400> 355
   aaactcctct atgcagtgta tcgaa 25
<210> 356
   <211> 25
   <212> DNA
   <213> Homo sapiens
<400> 356
   atagccatat ggggctggtc ctatg 25
<210> 357
   <211> 25
   <212> DNA
   <213> Homo sapiens
<400> 357
   tggaggatac gtttcatcac tggcc 25
<210> 358
   <211> 25
   <212> DNA
   <213> Homo sapiens
<400> 358
   ccttgcatct ggaactggtc ttttc 25
<210> 359
   <211> 25
   <212> DNA
   <213> Homo sapiens
<400> 359
   tggtatagca gtggctccag tctcc 25
<210> 360
   <211> 25
   <212> DNA
   <213> Homo sapiens
<400> 360
   gaatattacg cgtctgtcta cacag 25
<210> 361
   <211> 25
   <212> DNA
   <213> Homo sapiens
<400> 361
   gagagattca tgggtctccc aacaa 25
<210> 362
   <211> 25
   <212> DNA
   <213> Homo sapiens
<400> 362
   aatgtagact atcttctcat ccacg 25
<210> 363
   <211> 25
   <212> DNA
   <213> Homo sapiens
<400> 363
   tgaccagaac cacggcttat ccggc 25
<210> 364
   <211> 25
   <212> DNA
   <213> Homo sapiens
<400> 364
   acatgaccca cttcctaaag cagtg 25
<210> 365
   <211> 25
   <212> DNA
   <213> Homo sapiens
<400> 365
   gggagtttcc tgagggtttt ctcat 25
<210> 366
   <211> 25
   <212> DNA
   <213> Homo sapiens
<400> 366
   aaatgagggc tgcacattgc ctgtt 25
<210> 367
   <211> 25
   <212> DNA
   <213> Homo sapiens
<400> 367
   attgcctgtt ctgcttcgaa gtatt 25
<210> 368
   <211> 25
   <212> DNA
   <213> Homo sapiens
<400> 368
   gaaagcatat gcagccaacc aagat 25
<210> 369
   <211> 25
   <212> DNA
   <213> Homo sapiens
<400> 369
   aaagtcaccc aaacacttct gcttt 25
<210> 370
   <211> 25
   <212> DNA
   <213> Homo sapiens
<400> 370
   tgctttcact taagtgtctg gcccg 25
<210> 371
   <211> 25
   <212> DNA
   <213> Homo sapiens
<400> 371
   gtctggcccg caatactgta ggaac 25
<210> 372
   <211> 25
   <212> DNA
   <213> Homo sapiens
<400> 372
   aatatccaca gtactcactt tttcc 25
<210> 373
   <211> 25
   <212> DNA
   <213> Homo sapiens
<400> 373
   gtactcactt tttccaaatg atcct 25
<210> 374
   <211> 25
   <212> DNA
   <213> Homo sapiens
<400> 374
   gaaatatctt tctcttacct gttat 25
<210> 375
   <211> 25
   <212> DNA
   <213> Homo sapiens
<400> 375
   tatgtgccaa agctttacta ctgtg 25
<210> 376
   <211> 25
   <212> DNA
   <213> Homo sapiens
<400> 376
   aagtttggta tgtttctctc aagaa 25
<210> 377
   <211> 25
   <212> DNA
   <213> Homo sapiens
<400> 377
   aaagcatggt gagtccagac agcag 25
<210> 378
   <211> 25
   <212> DNA
   <213> Homo sapiens
<400> 378
   tggaaactcc ctgttagtac gtcct 25
<210> 379
   <211> 25
   <212> DNA
   <213> Homo sapiens
<400> 379
   ggtggttatt gatggcgttg ccttt 25
<210> 380
   <211> 25
   <212> DNA
   <213> Homo sapiens
<400> 380
   gttgcctttg tttcatcttt gagtt 25
<210> 381
   <211> 25
   <212> DNA
   <213> Homo sapiens
<400> 381
   tcatctttga gtttgccctt tgtgg 25
<210> 382
   <211> 25
   <212> DNA
   <213> Homo sapiens
<400> 382
   gtttgccctt tgtgggatct agtgg 25
<210> 383
   <211> 25
   <212> DNA
   <213> Homo sapiens
<400> 383
   gagcactgac agaactctta acagc 25
<210> 384
   <211> 25
   <212> DNA
   <213> Homo sapiens
<400> 384
   aacagcgtgc tgtatttttg acatt 25
<210> 385
   <211> 25
   <212> DNA
   <213> Homo sapiens
<400> 385
   tgacttgatt tgtacataac tctgt 25
<210> 386
   <211> 25
   <212> DNA
   <213> Homo sapiens
<400> 386
   gtttttgtac cttagaattt ctgca 25
<210> 387
   <211> 25
   <212> DNA
   <213> Homo sapiens
<400> 387
   ctactactat acggctgcga gaaga 25
<210> 388
   <211> 25
   <212> DNA
   <213> Homo sapiens
<400> 388
   atacggctgc gagaagacga cagaa 25
<210> 389
   <211> 25
   <212> DNA
   <213> Homo sapiens
<400> 389
   gacgacagaa gggctacgac gtggt 25
<210> 390
   <211> 25
   <212> DNA
   <213> Homo sapiens
<400> 390
   agaagggcta cgacgtggtg gcggt 25
<210> 391
   <211> 25
   <212> DNA
   <213> Homo sapiens
<400> 391
   ggtgtacccc aatgccaagg tggaa 25
<210> 392
   <211> 25
   <212> DNA
   <213> Homo sapiens
<400> 392
   gtaccccaat gccaaggtgg aactc 25
<210> 393
   <211> 25
   <212> DNA
   <213> Homo sapiens
<400> 393
   atgccaaggt ggaactcgtc tgagc 25
<210> 394
   <211> 25
   <212> DNA
   <213> Homo sapiens
<400> 394
   aggtggaact cgtctgagct gggac 25
<210> 395
   <211> 25
   <212> DNA
   <213> Homo sapiens
<400> 395
   ccctgctcgg ccggtgtaaa tatgt 25
<210> 396
   <211> 25
   <212> DNA
   <213> Homo sapiens
<400> 396
   tgctcggccg gtgtaaatat gtttc 25
<210> 397
   <211> 25
   <212> DNA
   <213> Homo sapiens
<400> 397
   gtgtaaatat gtttcaccct gtccc 25
<210> 398
   <211> 25
   <212> DNA
   <213> Homo sapiens
<400> 398
   aagacacttc ttccaaacct tgaat 25
<210> 399
   <211> 25
   <212> DNA
   <213> Homo sapiens
<400> 399
   gatgtgtgtt tacttcatgt ttaca 25
<210> 400
   <211> 25
   <212> DNA
   <213> Homo sapiens
<400> 400
   atcagccaaa accataactt acaat 25
<210> 401
   <211> 25
   <212> DNA
   <213> Homo sapiens
<400> 401
   ttggatatgc tttaccattc ttagg 25
<210> 402
   <211> 25
   <212> DNA
   <213> Homo sapiens
<400> 402
   accattctta ggtttctgtg gaaca 25
<210> 403
   <211> 25
   <212> DNA
   <213> Homo sapiens
<400> 403
   tttttccaat tgctattgcc caaga 25
<210> 404
   <211> 25
   <212> DNA
   <213> Homo sapiens
<400> 404
   gctattgccc aagaattgct ttcca 25
<210> 405
   <211> 25
   <212> DNA
   <213> Homo sapiens
<400> 405
   gaattgcttt ccatgcacat attgt 25
<210> 406
   <211> 25
   <212> DNA
   <213> Homo sapiens
<400> 406
   ttgtaaaaat tccgctttgt gccac 25
<210> 407
   <211> 25
   <212> DNA
   <213> Homo sapiens
<400> 407
   gctttgtgcc acaggtcatg attgt 25
<210> 408
   <211> 25
   <212> DNA
   <213> Homo sapiens
<400> 408
   agggactatt tgtattgtat gttgc 25
<210> 409
   <211> 25
   <212> DNA
   <213> Homo sapiens
<400> 409
   tcagggccaa gttcgtgggg acacc 25
<210> 410
   <211> 25
   <212> DNA
   <213> Homo sapiens
<400> 410
   gaccacctta taccagcgtt atgag 25
<210> 411
   <211> 25
   <212> DNA
   <213> Homo sapiens
<400> 411
   ggttccaagc cttaggggat gccgc 25
<210> 412
   <211> 25
   <212> DNA
   <213> Homo sapiens
<400> 412
   ggagagtgtc tgcggatact tccac 25
<210> 413
   <211> 25
   <212> DNA
   <213> Homo sapiens
<400> 413
   gtcccacaac cgcagcgagg agttt 25
<210> 414
   <211> 25
   <212> DNA
   <213> Homo sapiens
<400> 414
   aggatggact cttgcacatc actac 25
<210> 415
   <211> 25
   <212> DNA
   <213> Homo sapiens
<400> 415
   acatcactac ctgcagtttc gtggc 25
<210> 416
   <211> 25
   <212> DNA
   <213> Homo sapiens
<400> 416
   ccctggaaca gcctgagctt agctc 25
<210> 417
   <211> 25
   <212> DNA
   <213> Homo sapiens
<400> 417
   tgcacagtgt ttccctgttt atcca 25
<210> 418
   <211> 25
   <212> DNA
   <213> Homo sapiens
<400> 418
   tatccatccc ctgcaaactg cagag 25
<210> 419
   <211> 25
   <212> DNA
   <213> Homo sapiens
<400> 419
   ggcactcatt gcttgtggac ggacc 25

## Description

### BACKGROUND OF THE INVENTION

### FIELD OF THE INVENTION

The present invention relates to a method and a system for determining behavior of thyroid tumor.

### BACKGROUND

A disease in which nodules (tumors) are formed in the thyroid is generally referred to as "thyroid tumor". Thyroid tumors are benign in many cases. However, among the cases, there is a case of a malignant tumor called "thyroid carcinoma". In the diagnosis of thyroid tumors, the presence of thyroid tumors is confirmed by palpation, ultrasonography or the like. When the presence of thyroid tumors can be confirmed, in order to determine behavior (whether the tumors are benign or malignant), cells are collected from the tumors by fine-needle aspiration and cytological diagnosis is performed. However, the cytological diagnosis is performed based on the observation results of the cells and thus the determination results may vary depending on the experience and skill of a tester. Like the cases of follicular adenoma (benign tumor) and follicular carcinoma (malignant tumor), there are cases which have difficulty in being determined by even a skilled tester.

In the test for thyroid carcinoma, there is also a blood test for examining thyroid hormone or tumor markers in the blood. For example, it is known that when the thyroid carcinoma is medullary carcinoma, the value of calcitonin in thyroid hormone and the value of tumor marker CEA are increased. However, if the symptoms of thyroid carcinoma are shown, the value of the tumor marker is not always increased. Thus, thyroid carcinoma cannot be diagnosed only by the results of the blood test.

On the other hand, methods based on gene expression information have been recently studied and developed as new methods for determining behavior of thyroid tumor. For example, a microarray called Afirma (registered trademark) Thyroid FNA Analysis from Veracyte, Inc. is currently commercially available. Further, Alexander EK. et al. (Preoperative Diagnosis of Benign Thyroid Nodules with Indeterminate Cytology. NEng J Med, 2012; 367: 705-715)) discloses the results obtained by determining behavior of thyroid tumor using the Thyroid FNA Analysis. Specifically, as for specimens which are not determined to be either benign or malignant in cytodiagnosis (n=265), expression information of 142 genes is analyzed using Thyroid FNA Analysis to classify them into benignancy or suspicion of malignancy. Further, the classified results are compared with the results based on histopathological diagnosis. In the histopathological diagnosis, 85 specimens of 265 specimens are determined to be malignant. In the gene expression analysis, 78 specimens of the 85 specimens are determined to be suspicious of malignancy. That is, as the classification performance, the sensitivity is 92%, the specificity is 52%, the positive predictive value (PPV) is 47%, and the negative predictive value (NPV) is 93%. If performance when follicular neoplasms which are particularly difficult to determine (n=105) are classified into a follicular adenoma (benign) and follicular carcinoma (malignant) in the cytological diagnosis is calculated, the sensitivity is 95%, the specificity is 48%, the PPV is 30%, and the NPV is 98%.

Determining behavior of thyroid tumor based on the analysis results of the gene expression is an objective means and it is considered to be useful for the tumors which are difficult to determine. However, in the case of the determination using the currently commercially available Thyroid FNA Analysis, the specificity and PPV are very low as described above. On the other hand, the thyroid tumors are benign in many cases. Therefore, in the determination method in which the specificity and PPV are low, benign tumors in many subjects are mistakenly determined to be malignant in practice. As a result, there is a possibility of performing unnecessary medical treatment on the subjects with benign tumors.

WO 2010/030365 A2 discusses a method for detecting whether thyroid cancer cells are present in a tissue, based on differential expression of certain genes.

### SUMMARY OF THE INVENTION

The scope of the present invention is defined solely by the appended claims, and is not affected to any degree by the statements within this summary.

The present inventors have exhaustively analyzed the expression levels of genes in diseased tissues with follicular adenoma and diseased tissues with follicular carcinoma. As a result, the present inventors have identified a gene cluster in which the expression is increased in benign and malignant tumors as a novel marker set. The present inventors have found that it is possible to determine whether thyroid tumor is a benign or malignant tumor based on the measured expression level of the marker set and it is possible to determine whether the malignant tumor is a tumor with poor prognosis, and have completed the present invention.

That is, the present invention provides a system adapted to a method for determining behavior of thyroid tumor as defined in claim 8. The system comprises a processor, and a memory, under control of said processor, including software instructions adapted to enable the system to perform operations. The operations comprise:
determining whether a thyroid tumor in a subject is benign or malignant based on the results of first, second, and third measurements;
wherein the expression levels of at least 6 genes selected from the group consisting of AIF1L, CDH16, FAM162B, FGFR2, GJB6, KCNJ13, KIAA1467, SLC25A15, TFCP2L1, TFF3, and TMEM171 in a sample collected from the subject are measured in the first measurement,
the expression levels of at least 7 genes selected from the group consisting of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18 in the sample are measured in the second measurement, and
the expression levels of at least 7 genes selected from the group consisting of ANXA1, C13orf33, CYP1B1, FAP, FN1, IL17RD, PDLIM4, RUNX2, and TIMP1 in the sample are measured in the third measurement.

Further, disclosed is a computer program product for enabling a computer to determine behavior of thyroid tumor. The product comprises a computer readable medium, and software instructions, on the computer readable medium, for enabling the computer to perform predetermined operations. The operations comprises:
determining whether a thyroid tumor in a subject is benign or malignant based on the results of first, second, and third measurements, wherein
the expression levels of at least 6 genes selected from the group consisting of AIF1L, CDH16, FAM162B, FGFR2, GJB6, KCNJ13, KIAA1467, SLC25A15, TFCP2L1, TFF3, and TMEM171 in a sample collected from the subject are measured in the first measurement,
the expression levels of at least 7 genes selected from the group consisting of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18 in the sample are measured in the second measurement,
and the expression levels of at least 7 genes selected from the group consisting of ANXA1, C13orf33, CYP1B1, FAP, FN1, IL17RD, PDLIM4, RUNX2, and TIMP1 in the sample are measured in the third measurement.

Further, the present invention provides a method for determining behavior of thyroid tumor as defined in claim 1. The method comprises:
applying a sample collected from a subject with a thyroid tumor to a microarray capable of measuring the expression levels of the following genes (a) to (c):
   (a) at least 6 genes selected from the group consisting of AIF1L, CDH16, FAM162B, FGFR2, GJB6, KCNJ13, KIAA1467, SLC25A15, TFCP2L1, TFF3, and TMEM171;
   (b) at least 7 genes selected from the group consisting of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18; and
   (c) at least 7 genes selected from the group consisting of ANXA1, C13orf33, CYP1B1, FAP, FN1, IL17RD, PDLIM4, RUNX2, and TIMP1;
detecting optical information about the expression levels of the genes (a) to (c) obtained from the microarray using a microarray scanner after the applying step;
sending the optical information detected in the detecting step to a CPU of a computer connected to the microarray scanner;
calculating the expression levels of the genes (a) to (c), based on the optical information by the CPU; and
determining whether the thyroid tumor in the subject is benign or malignant using the values calculated by the CPU in the calculating step.

According to the method for determining behavior of thyroid tumor of the present invention (hereinafter, also referred to as "determination method"), it is possible to determine whether thyroid tumor is a benign or malignant tumor with high accuracy.

According to the method for determining behavior of thyroid tumor of the present invention (hereinafter, also referred to as "determination method"), it is possible to determine whether thyroid tumor is a benign or malignant tumor with high accuracy.

### BRIEF DESCRIPTION OF THE DRAWINGS

Fig. 1 shows the results of cluster analysis of the expression levels of 37 genes obtained by measuring specimens with follicular neoplasm (Learning set) with a microarray;
Fig. 2 shows the results of cluster analysis of the expression levels of 37 genes obtained by measuring specimens with follicular neoplasm (Validation set) with a microarray;
Fig. 3 shows the results of cluster analysis of the expression levels of 37 genes obtained by measuring specimens with thyroid tumors other than follicular neoplasm with a microarray;
Fig. 4 shows a determination system of this embodiment;
Fig. 5 is a block diagram showing a functional configuration of the determination system shown in Fig. 4;
Fig. 6 is a block diagram showing a hardware configuration of the determination system shown in Fig. 4; and
Fig. 7 is a flow chart of the determination using the determination system shown in Fig. 4.
Fig. 8 is a table showing the determination according to the present invention.

### DETAILED DESCRIPTION OF THE PREFERRED EMBODIMENTS

The preferred embodiments of the present invention will be described hereinafter with reference to the drawings.

In the determination method of the present invention, as for biological samples collected from subjects with thyroid tumors, the expression levels of at least 6 genes selected from a first gene cluster, the expression levels of at least 7 genes selected from a second gene cluster, and the expression levels of at least 7 genes selected from a third gene cluster are measured as further defined by claim 1.

The term "thyroid tumors" used herein means nodules (tumors) formed in the thyroid and intends to include both benign and malignant tumors. In the art, the thyroid tumors are also referred to as "thyroid nodules" or "adenomatous goiter".

The term "determining behavior of thyroid tumor" means determining whether the thyroid tumor is benign or malignant. When the thyroid tumor is malignant, it can include determining whether the tumor is a malignant tumor with poor prognosis or a malignant tumor with good prognosis.

Here, the term "malignant tumor with poor prognosis" means a malignant tumor which has a high degree of malignancy and a high possibility of recurrence and/or metastasis among the malignant thyroid tumors and thus has a high mortality rate in patients. Further, the term "malignant tumor with good prognosis" means a malignant tumor which has a low degree of malignancy and a low possibility of recurrence and/or metastasis among the malignant thyroid tumors and thus has a low mortality rate in patients. The malignant tumor with poor prognosis largely differs in therapeutic strategies from the malignant tumor with good prognosis. Consequently, distinction between the malignant tumor with poor prognosis and the malignant tumor with good prognosis becomes useful information in determination of therapeutic strategies.

The term "subjects with thyroid tumors" used herein means subjects who are confirmed to have thyroid tumors or strongly suspected to have thyroid tumors by palpation or ultrasonography.

In this embodiment, the biological sample is not particularly limited as long as it is a sample collected from thyroid lesion tissues of the subject, such as a sample derived from a living body which contains cells derived from the lesion site in the thyroid. Examples of a sample derived from a living body as disclosed herein include blood, serum, plasma, lymph, cells collected by fine-needle aspiration, and tissues collected by surgery. Further, cultures obtained by culturing the cells and tissues extracted from subjects may also be used.

The term "gene cluster" used herein means a group consisting of plural genes.

The term "gene expression level" used herein means an amount of RNA transcribed from each gene or an amount of material reflecting the amount. Therefore, in the embodiment of the present invention, a nucleic acid (particularly RNA) is extracted from a biological sample. The amount of RNA derived from each gene contained in the nucleic acid or the amount of cRNA obtained by IVT-amplification of cDNA or cDNA obtained by reverse transcription of the RNA is measured as the gene expression level. The amount of RNA transcribed from each gene is small in many cases. Accordingly, it is preferable to measure the amount of cDNA obtained from the RNA or the amount of cRNA.

A method for extracting a nucleic acid including RNA from a biological sample is known in the art. RNA can be extracted from a biological sample by, for example, a method comprising mixing the biological sample with a treatment solution containing a surfactant which solubilizes cells or tissues (sodium cholate, sodium dodecyl sulfate etc.), physically treating (agitation, homogenization, ultrasonication etc.) the resulting mixture to release RNA contained in the biological sample into the mixed solution. Preferably, the mixed solution containing the released RNA is centrifuged to recover a supernatant, and the supernatant is extracted with phenol/chloroform to purify the RNA. The extraction and purification of RNA from the biological sample can be performed using a commercially available kit.

In the art, the base sequences themselves of the respective genes are known. These base sequences can be obtained from, for example, a known database (http://www.ncbi.nlm.nih.gov/ (provided by National Center for Biotechnology Information (NCBI)). The NCBI Accession Nos and Probe IDs of the genes, and Seq ID Nos. of oligonucleotide probes included in each probe set are shown in Table 1. The IDs are up to date as of November 16, 2012. Probe ID is a number for identifying probes on GeneChip (registered trademark) Human Genome U133 Plus 2.0 Array (Affymetrix, Inc.). The base sequences of probes can be obtained from, for example, the website of Affymetrix, Inc. (http://www.affymetrix.com/analysis/index.affx).

**[Table 1]**

| Symbol | Gene IDs (NCBI) | Probe Set ID | Seq ID NOs |
|---|---|---|---|
| AIF1L | NM_031426 | 223075_s_at | 1-11 |
| CDH16 | NM_004062 | 206517_at | 12-22 |
| FAM162B | NM_001085480 | 228875_at | 23-33 |
| FGFR2 | NM_000141, NM_001144913, NM_001144914, NM_001144915, NM_001144916, NM_001144917, NM_001144918, NM_001144919, NM_022970 | 203638_s_at | 34-44 |
| GJB6 | NM_001110219, NM_001110220, NM_001110221, NM_006783 | 231771_at | 45-55 |
| KCNJ13 | NM_001172416, NM_001172417, NM_002242 | 210179_at | 56-66 |
| KIAA1467 | NM_020853 | 213234_at | 67-77 |
| SLC25A15 | NM_014252 | 218653_at | 78-88 |
| TFCP2L1 | NM_014553 | 227642_at | 89-99 |
| TFF3 | NM_003226 | 204623_at | 100-110 |
| TMEM171 | NM_001161342.1, NM_173490 | 240770_at | 111-121 |
| C4orf10 | NR_015453 | 214123_s_at | 122-132 |
| CCDC8 | NM_032040 | 223495_at | 133-143 |
| CD22 | NM_001771 | 38521_at | 144-159 |
| FAM125A | NM_138401 | 227864_s_at | 160-170 |
| FAM174B | NM_207446 | 51158_at | 171-186 |
| FBF1 | NM_001080542 | 1555288_s_at | 187-197 |
| GLB1L2 | NM_138342 | 213713_s_at | 198-208 |
| LOC644613 | BC025792 | 1563426_a_at | 209-219 |
| MAP2K2 | NM_030662 | 202424_at | 220-230 |
| MTG1 | NM_138384 | 212767_at | 231-241 |
| PFKL | NM_002626, NR_024108 | 211065_x_at | 242-252 |
| PTDSS2 | NM_030783 | 221005_s_at | 253-263 |
| SF3A2 | NM_007165 | 37462_i_at | 264-276 |
| SLC2A11 | NM_001024938, NM_001024939, NM_030807 | 232167_at | 277-287 |
| VILL | NM_015873 | 209950_s_at | 288-298 |
| VSIG2 | NM_014312 | 228232_s_at | 299-309 |
| WDR18 | NM_024100 | 209461_x_at | 310-320 |
| ANXA1 | NM_000700 | 201012_at | 321-331 |
| C13orf33 | NM_032849 | 227058_at | 332-342 |
| CYP1B1 | NM_000104 | 202437_s_at | 343-353 |
| FAP | NM_004460 | 209955_s_at | 354-364 |
| FN1 | NM_002026, NM_054034, NM_212474, NM_212475, NM_212476, NM_212478, NM_212482 | 211719_x_at | 365-375 |
| IL17RD | NM_017563 | 227997_at | 376-386 |
| PDLIM4 | NM_001131027, NM_003687 | 214175_x_at | 387-397 |
| RUNX2 | NM_001015051, NM_001024630, NM_004348 | 232231_at | 398-408 |
| TIMP1 | NM_003254 | 201666_at | 409-419 |

The 37 genes are a cluster of genes in which the fact that the expression in cases with benign and malignant tumors is increased is found out by the present inventors. Specifically, the first gene cluster is a cluster of genes in which an increase in the expression of the cases with benign tumors is more than that of the cases with malignant tumors. In addition, the second and third gene clusters are a cluster of genes in which an increase in the expression of the cases with malignant tumors is more than that of the cases with benign tumors. Further, the present inventors have found that the expression of the second gene cluster is increased, particularly in the cases of malignant tumors with poor prognosis, and the expression of the third gene cluster is increased, particularly in the cases of malignant tumors with good prognosis.

In the embodiment of the present invention, there is no particular restriction to measure the expression level of any of the genes of the first, second, and third gene clusters, and it can be arbitrarily selected. As an example, a method for measuring the expression level of at least 6 genes: AIF1L, FAM162B, FGFR2, GJB6, KIAA1467, and TFF3 of the first gene cluster, the expression level of at least 7 genes: FAM174B, MAP2K2, MTG1, PFKL, PTDSS2, SF3A2, and WDR18 of the second gene cluster, and the expression level of at least 7 genes: ANXA1, CYP1B1, FAP, IL17RD, PDLIM4, RUNX2, and TIMP1 of the third gene cluster is listed. However, the determination method of the present invention is not limited to the example.

In the embodiment of the present invention, the method for measuring the gene expression level is not particularly limited and it can be selected from any known method in the art. A measurement method using a microarray or a nucleic acid amplification method is preferred.

The microarray suitable for the determination method of the present invention is not particularly limited as long as it is a chip in which a nucleic acid probe having about 20 to 25 nucleotides which can specifically hybridize to cDNA or cRNA derived from RNAs transcribed from the genes or the RNAs (hereinafter, also referred to as "target nucleic acid molecule") is immobilized on an appropriate substrate. The probe can be appropriately designed based on the base sequences of the genes. Further, the microarray can be produced by any known method in the art. In the embodiment of the present invention, a commercially available microarray may be used as long as the probe which can specifically hybridize to the target nucleic acid molecule is mounted. Examples of the microarray include GeneChip (registered trademark, manufactured by Affymetrix, Inc.).

As used herein, the term "can specifically hybridize" means that a probe can hybridize to a target nucleic acid molecule under a stringent condition. The term "stringent condition" means a condition under which the probe can hybridize to the target nucleic acid molecule with a detectably higher extent than it does to a nucleic acid molecule other than the target nucleic acid molecule (e.g. more than at least two times of the background). The stringent condition generally depends on the sequences and varies depending on various circumstances. Generally, the stringent condition is selected so that it is about 5°C lower than a thermal melting point (Tm) of the specific sequence under a certain ionic strength and pH. This Tm is a temperature at which 50% of the complementary probe hybridizes to the base sequence of the target nucleic acid molecule in equilibrium (under a certain ionic strength, pH and nucleic acid composition). Such condition may be those which are used in hybridization techniques between polynucleotides known in the art. Specifically, it may be a condition of pH 7.0 to 9.0, a salt concentration of lower than about 1.5 M Na-ion, more specifically about 0.01 to 1.0 M Na-ion concentration (or other salt), and a temperature of at least about 30°C. For example, the stringent condition in a microarray technique includes the hybridization at 37°C in 50% formamide, 1 M NaCl, and 1% SDS; and washing at 60 to 65°C in 0.1×SSC.

When the microarray technique is used, the target nucleic acid molecule is preferably labeled with a labeling substance known in the art. Labeling of the target nucleic acid molecule facilitates measurement of a signal from a probe on a microarray. In the embodiment of the present invention, RNA extracted from a biological sample may be labeled. Preferably, cDNA or cRNA derived from the RNA is labeled. Examples of the labeling substance include fluorescent substances, haptens such as biotin, and radioactive substances. Examples of the fluorescent substances include Cy3, Cy5, FITC, Alexa Fluor (trademark). A method for labeling RNA, cDNA, and cRNA with these labeling substances is known in the art.

In the measurement using a microarray, the gene expression level is obtained as a signal from a probe, such as fluorescence intensity, luminescence intensity or amount of current. Each of the signals may be detected using a scanner installed in a general microarray analyzer. Examples of the scanner include GeneChip (registered trademark) Scanner 3000 7G (Affymetrix, Inc.) and Illumina (registered trademark) Bead Array Reader (Illumina).

Examples of the nucleic acid amplification method suitable for the determination method of the present invention include a polymerase-chain-reaction (PCR) assay, a strand displacement activity assay, a ligase-chain-reaction assay, and a transcriptional amplification method. These nucleic acid amplification methods themselves are known in the art. As the PCR assay, for example, a real-time RT-PCR assay is listed. As the strand displacement activity assay, for example, a real-time RT-LAMP assay (refer to, for example, US6410278) is listed. As the transcriptional amplification method, for example, a TAS assay is listed. Among these methods, the real-time RT-PCR assay and the real-time RT-LAMP assay are preferred.

The primer to be used for a nucleic acid amplification method is not particularly limited as long as it is a primer which can be amplified by specific hybridization to the target nucleic acid molecule. The stringent condition in the PCR assay may be a condition of pH 7.0 to 9.0, 0.01 to 0.1 M Tris HCl, 0.05 to 0.15 M K-ion concentration (or other salt), and a temperature of at least about 55°C. Such a primer can be appropriately designed based on the base sequences of the respective genes. Further, the primer is preferably designed according to the type of the nucleic acid amplification method. The length of the primer is usually from 5 to 50 nucleotides, preferably from 10 to 40 nucleotides. The primer can be produced by any nucleic acid synthesis method known in the art.

The primer may be labeled with any labeling substance known in the art. Labeling of the primer may be performed using radioactive elements or non-radioactive molecules. Examples of the radioactive isotope include ³²P, ³³P, ³⁵S, ³H, and ¹²⁵I. Examples of the non-radioactive substance include ligands such as biotin, avidin, streptoavidin, and digoxigenin; hapten, pigment, and luminescent reagents such as chemiluminescent, bioluminescent, fluorescent and phosphorescent reagents.

The reaction condition of the nucleic acid amplification method varies depending on the type of the nucleic acid amplification method and the base sequences of the primer, and it can be appropriately set with reference to, for example, a method described in Molecular Cloning: A Laboratory Manual (2nd ed.) (Sambrook, J. et al. Cold Spring Harbor Laboratory Press, New York (1989)).

The gene expression level is measured as described above, followed by determining whether the thyroid tumor in the subject with thyroid tumor is benign or malignant based on the measured expression levels of genes of the first, second, and third gene clusters in the determination method of the present invention. Here, the benign tumor is not particularly limited as long as it is a thyroid tumor known to be benign in the art, and it preferably means at least one of follicular adenoma, adenomatous nodules, and cysts. Further, the malignant tumor is not particularly limited as long as it is a thyroid tumor known to be malignant in the art, and it preferably means at least one of follicular carcinoma, papillary carcinoma, poorly-differentiated carcinoma, and undifferentiated carcinoma. The malignant tumor includes a malignant tumor with poor prognosis and a malignant tumor with good prognosis.

In the embodiment of the present invention, the expression states of the gene clusters are compared based on the measured expression levels of genes of the first, second, and third gene clusters and the determination is performed based on the results. That is, when the expression state of the genes measured in the first measurement is more than or equal to the expression state of the genes measured in the second measurement, and when the expression state of the genes measured in the first measurement is more than or equal to the expression state of the genes measured in the third measurements, the thyroid tumor of the subject is determined to be benign. On the contrary, when the expression state of the genes measured in either the second or third measurement is more than the expression state of the genes measured in the first measurement, the thyroid tumor of the subject is determined to be malignant.

In the present invention, the term "expression state of genes selected from the gene cluster" means an indicator of the level or tendency of the gene expression in a plurality of genes selected from predetermined gene clusters. As the expression state, for example, a measure of central tendency of the expression levels (measured value) in a plurality of selected genes is listed. The measure of central tendency is not particularly limited as long as it is a statistics value which summarizes the distribution of the gene expression in a plurality of genes selected from predetermined gene clusters. Examples of the measure of central tendency include an average, a median, a maximum, and a mode. Among them, the average is particularly preferred. The term "the expression state is increased" means a measure of central tendency of the expression levels in a plurality of genes selected from certain gene clusters is higher than that of the expression levels in a plurality of genes selected from the remaining gene clusters. Therefore, when an average of the expression levels is used as an expression state of genes selected from a gene cluster, comparison of the expression states is performed as follows. As for biological samples of subjects, the sum of the expression levels of at least 6 genes selected from the first gene cluster is calculated. The calculated value is divided by the number of the selected genes to obtain an average. The same calculation is performed on at least 7 genes selected from the second and third gene clusters. Then, the obtained three averages are compared. For example, when the average in the genes selected from the first gene cluster is the highest, the thyroid tumor of the subject is determined to be benign. On the contrary, when the average in the genes selected from the second or third gene cluster is the highest, the thyroid tumor of the subject is determined to be malignant.

In the embodiment of the present invention, the expression state of genes selected from each of the gene clusters in biological samples of subjects may be obtained by using the expression level data previously obtained as for the cases with benign and malignant tumors in which behavior is determined by pathological diagnosis (control samples). For example, the multivariate analysis is performed using the expression level data previously obtained as for the control samples so that a formula which reflects or predicts the expression states of genes selected from the gene clusters can be obtained. The information on the expression states of the gene clusters in the biological samples of the subjects is obtained by using the obtained formula. Based on the information, the samples can be determined. Preferable examples of the multivariate analysis include discriminant analysis. Examples of the discriminant analysis include Fischer's linear discriminant analysis, Mahalanobis distance-based discriminant analysis, Euclid distance-based discriminant analysis, Multi-group discriminant analysis, variable selection, and canonical discriminant analysis. Among them, particularly the Euclid distance-based discriminant analysis is preferred. The Euclid distance shows similarity in gene expression patterns among the control samples and the biological samples of the subjects. The Euclid distance can be obtained as follows. Based on the expression level data of the control samples which is previously obtained, the control samples are first classified into three groups by the cluster analysis known in the art. Conveniently the three groups are referred to as "Cluster 1" with predominant expression of genes selected from the first gene cluster, "Cluster 2" with predominant expression of genes selected from the second gene cluster, and "Cluster 3" with predominant expression of genes selected from the third gene cluster, respectively. Subsequently, the data of the expression levels of the groups is plotted to calculate an average of the groups. Then, as for the average of the groups and the expression level data obtained from the biological samples of the subjects, Euclid distances among the control samples and the biological samples of the subjects are calculated.

When the Euclid distance is used, the term "expression state is increased" means that a Euclid distance between data of a certain cluster and the data of the subject is closer than an Euclid distance between data of the remaining clusters and the data of the subject. Therefore, comparison of the expression states is performed as follows. Three Euclid distances calculated are compared. For example, when the distance from Cluster 1 is the closest, the thyroid tumor of the subject is determined to be benign. On the contrary, when the distance from Cluster 2 or Cluster 3 is the closest, the thyroid tumor of the subject is determined to be malignant.

In another embodiment of the present invention, the expression states of genes selected from the gene clusters are compared based on the measured expression levels of genes of the first, second, and third gene clusters. Based on the results, behavior of thyroid tumor in a subject can be determined as follows.

That is, when the expression state of the genes measured in the first measurement is more than or equal to the expression state of the genes measured in the second measurement, and when the expression state of the genes measured in the first measurement is more than or equal to the expression state of the genes measured in the third measurement, the thyroid tumor of the subject is determined to be benign.

When the expression state of the genes measured in the second measurement is more than the genes measured in the first measurement, and when the expression state of the genes measured in the second measurement is more than or equal to the expression state of the genes measured in the third measurement, the thyroid tumor of the subject is determined to a first malignant tumor.

When the expression state of the genes measured in the third measurement is more than the expression states of the genes measured in the first and second measurements, the thyroid tumor of the subject is determined to a second malignant tumor.

The term "first malignant tumor" used herein means a malignant tumor with poor prognosis and the term "second malignant tumor" means a malignant tumor with good prognosis. The malignant tumor with poor prognosis is not particularly limited as long as it is a malignant thyroid tumor which is known to be poor prognosis in the art. Preferable examples thereof include at least one of undifferentiated carcinoma, poorly-differentiated carcinoma, and follicular carcinoma with widely invasive and/or vascular invasion. The malignant tumor with poor prognosis is not particularly limited as long as it is a malignant thyroid tumor which is known to be poor prognosis in the art. Preferable examples thereof include at least one of papillary carcinoma and follicular carcinoma without widely invasive and vascular invasion.

A marker set for determining behavior of thyroid tumor (hereinafter also referred to as "marker set") is included in the scope of the present invention. The marker set of the present invention includes at least 6 genes selected from the first gene cluster, at least 7 genes selected from the second gene cluster, and at least 7 genes selected from the third gene cluster.

In the embodiment of the present invention, there is no particular restriction to use any of the genes of the first, second, and third gene clusters as a marker, and it can be arbitrarily selected as long as a marker set including at least 6 genes: AIF1L, FAM162B, FGFR2, GJB6, KIAA1467, and TFF3 of the first gene cluster, at least 7 genes: FAM174B, MAP2K2, MTG1, PFKL, PTDSS2, SF3A2, and WDR18 of the second gene cluster, and at least 7 genes: ANXA1, CYP1B1, FAP, IL17RD, PDLIM4, RUNX2, and TIMP1 of the third gene cluster is used. However, the marker set disclosed herein is not limited to the example.

In the embodiment of the present invention, the expression levels of genes in a sample containing RNAs prepared from the biological sample collected from the subject is analyzed and the behavior of thyroid tumor in the subject can be determined based on the obtained expression levels. The measurement and analysis of the expression levels of the markers are the same as described above.

A kit for determining behavior of thyroid tumor (hereinafter, also referred to as "kit of the present invention") is also disclosed herein. That is, the present disclosure provides the kit for determining behavior of thyroid tumor including a probe set or primer set for measuring the expression levels of at least 6 genes selected from the first gene cluster composed of AIF1L, CDH16, FAM162B, FGFR2, GJB6, KCNJ13, KIAA1467, SLC25A15, TFCP2L1, TFF3, and TMEM171, the expression levels of at least 7 genes selected from the second gene cluster composed of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18, and the expression levels of at least 7 genes selected from the third gene cluster composed of ANXA1, C13orf33, CYP1B1, FAP, FN1, IL17RD, PDLIM4, RUNX2, and TIMP1.

The kit of the present disclosure can be suitably used for the determination method of the present invention. The probe and primer sets included in the kit of the present disclosure are the same described in the probe or primer which can specifically hybridize to the target nucleic acid molecule. The kit of the present disclosure may be in the form of microarray in which the probe is immobilized on an appropriate substrate.

In the disclosure herein, there is no particular restriction to include any of the probes or primer for measuring the gene expression in the kit, and it can be arbitrarily selected. As an example, a kit including a probe set or primer set for measuring at least the expression levels of AIF1L, FAM162B, FGFR2, GJB6, KIAA1467, and TFF3 of the first gene cluster, at least the expression levels of FAM174B, MAP2K2, MTG1, PFKL, PTDSS2, SF3A2, and WDR18 of the second gene cluster, and at least the expression levels of ANXA1, CYP1B1, FAP, IL17RD, PDLIM4, RUNX2, and TIMP1 of the third gene cluster is listed. However, the kit of the present disclosure is not limited to the example.

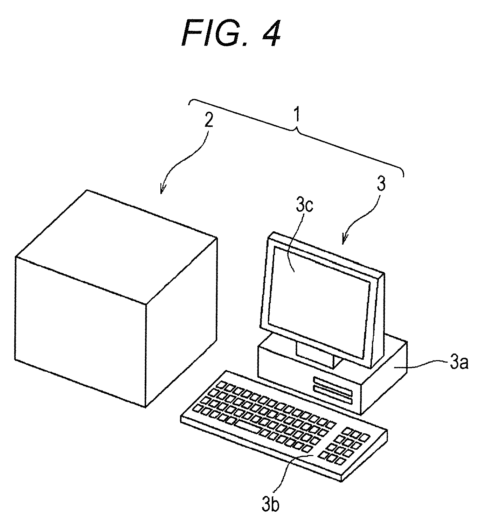

The determination of behavior of thyroid tumor can be performed by, for example, the determination system 1 shown in Fig. 4. Hereinafter, the present invention will be more specifically described with reference to the attached drawings; however, the present invention is not limited to the embodiment.

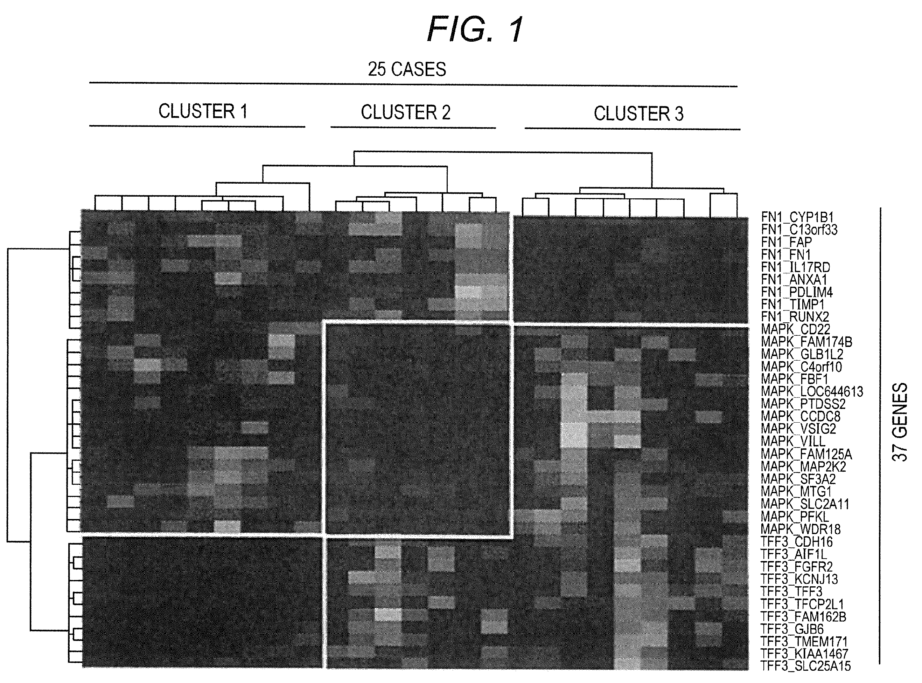

Fig. 1 is an outline explanatory view of a system according to one embodiment of the present invention. The determination system 1 shown in Fig. 1 includes a measurement device 2 and a computer system 3 connected to the measurement device 2.

In this embodiment, the measurement device 2 is a microarray scanner that detects a signal based on the hybridization of the probe and the target nucleic acid molecule on a microarray. The measurement device 2 detects signals corresponding to expression levels of genes selected from a first gene cluster, a second gene cluster, and a third gene cluster. Specifically, in this embodiment, the signals represent optical information. As the optical information, for example, a fluorescent signal is listed; however, the present invention is not limited only thereto. In this case, when the microarray after contact with the target nucleic acid molecule is set in the measurement device 2, the measurement device 2 obtains optical information based on the target nucleic acid molecule bound to the probe on the microarray and sends the obtained optical information to the computer system 3.

Any microarray scanner may be used as long as it can detect a signal based on the target nucleic acid molecule. The signal varies depending on the labeling substance used for labeling cDNA or cRNA in the measurement sample. Thus, as the microarray scanner, one suitable for detecting the signal from the labeling substance can be appropriately selected according to the type of the labeling substance. For example, when the labeling substance is a radioactive substance, a microarray scanner which can detect radioactive rays from the radioactive substance can be used as the measurement device 2.

When the gene expression level is detected by the nucleic acid amplification method, the measurement device 2 may be a nucleic acid amplification detector. In this case, a reaction solution containing DNA from a biological sample, an enzyme for nucleic acid amplification, and a primer is set in the measurement device 2. The nucleic acid in the reaction solution is amplified by the nucleic acid amplification method. The measurement device 2 obtains optical information such as fluorescence generated from the reaction solution by the amplification reaction or turbidity of the reaction solution and sends the optical information to the computer system 3.

The computer system 3 includes a computer main body 3a, an input device 3b, and a display unit 3c that displays specimen information, determination results, and the like. The computer system 3 receives optical information from the measurement device 2. The processor of the computer system 3 executes a program that determines behavior of thyroid tumor based on the optical information.

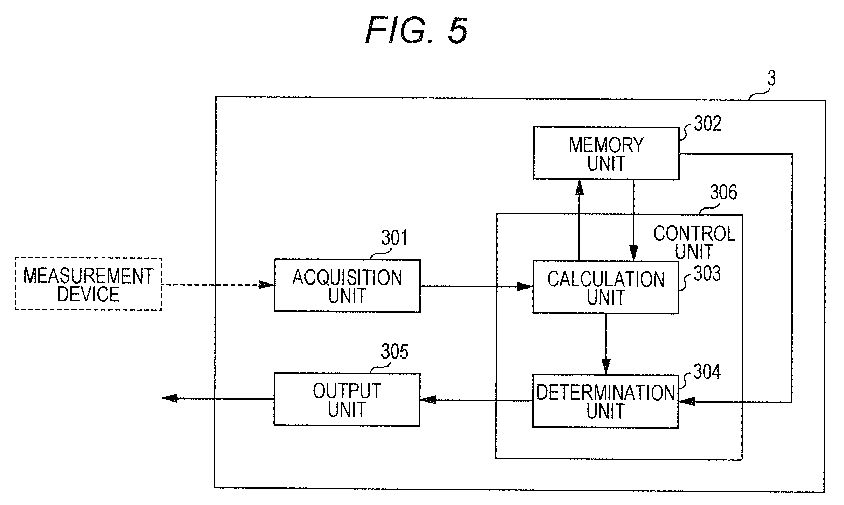

Fig. 5 is a block diagram showing a functional configuration of the determination system shown in Fig. 4.

The computer system 3 comprises an acquisition unit 301, a memory unit 302, a calculation unit 303, a determination unit 304, and an output unit 305 as shown in Fig. 5. The acquisition unit 301 is communicably connected to the measurement device 2 via a network. The calculation unit 303 and the determination unit 304 form a control unit 306.

The acquisition unit 301 obtains information sent from the measurement device 2.

The memory unit 302 stores a program that distinguishes behavior of thyroid tumor.

The calculation unit 303 calculates fluorescence intensity etc. using information obtained by the acquisition unit 301 and analyzes the gene expression state. As described above, as the gene expression state, for example, a measure of central tendency of the expression levels (measured values) in a plurality of selected genes is listed.

The determination unit 304 determines behavior of thyroid tumor based on the gene expression state analyzed by the calculation unit 303 and determination criteria stored in the memory unit 302.

The output unit 305 outputs determination results by the determination unit 304.

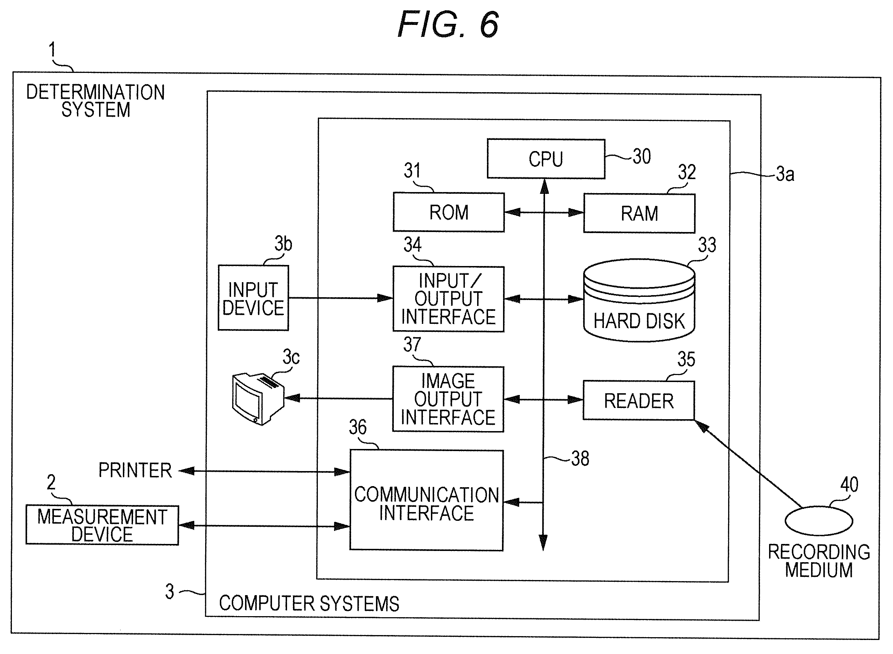

Fig. 6 is a block diagram showing a hardware configuration of the determination system shown in Fig. 4.

As shown in Fig. 6, the computer main body 3a comprises a CPU (Central Processing Unit) 30, a ROM (Read Only Memory) 121, a ROM 32, a hard disk 33, an input/output interface 34, a read-out device 35, a communication interface 36, and an image output interface 37. The CPU 30, the ROM 31, the RAM (Random Access Memory) 32, the hard disk 33, the input/output interface 34, the read-out device 35, the communication interface 36, and the image output interface 37 are data-communicably connected by a bus 38.

The CPU 30 is capable of executing the computer programs stored in the ROM 31 and the computer programs loaded in the ROM 32. The CPU 30 executes an application program to realize the blocking of the functions. Thus, the computer system functions as a terminal of a system for determining behavior of thyroid tumor.

The ROM 31 is configured by mask ROM, PROM, EPROM, EEPROM, and the like. Computer programs to be executed by the CPU 30 and data used for the same are recorded in the ROM 31.

The ROM 32 is configured by SRAM, DRAM, and the like. The ROM 32 is used to read out the computer programs recorded on the ROM 31 and the hard disc 33. In executing the computer program, the ROM 32 is used as a work region of the CPU 30.

The hard disk 33 is installed with an operating system to be executed by the CPU 30, a computer program such as an application program (computer program for determining the presence or absence of sensitivity to preoperative chemotherapy on breast cancer), and data used to execute the computer program.

The read-out device 35 is configured by a flexible disk drive, a CD-ROM drive, a DVD-ROM drive, and the like. The read-out device 35 is able to read out computer programs or data recorded on a portable recording medium 40.

The input/output interface 34 includes a serial interface such as USB, IEEE1394, and RS-232C; a parallel interface such as SCSI, IDE, and IEEE1284; and an analog interface such as D/A converter and A/D converter. The input/output interface 34 is connected to the input device 3b such as a keyboard and a mouse. Operators can use the input device 3b to input data into the computer main body 3a.

The communication interface 36 is, for example, an Ethernet (registered trademark) interface. The computer system 3 can receive optical information from the measurement device 2 through the communication interface 36 and send print data to a printer.

The image output interface 37 is connected to the display unit 3c configured by LCD, CRT, or the like. Thus, the display unit 3c can output an image signal corresponding to the image data provided from the CPU 30. The display unit 3c displays an image (screen) according to the input image signal.

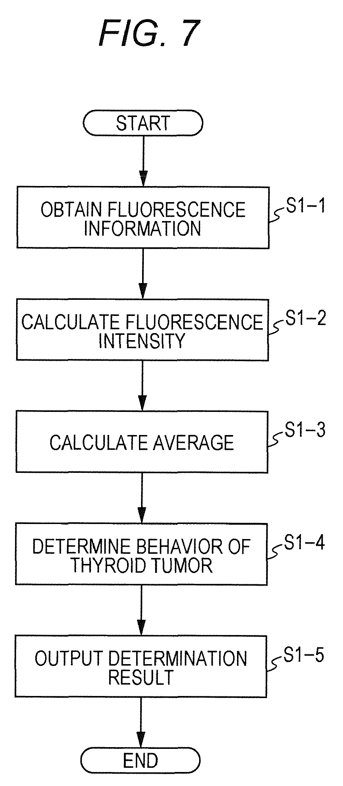

Subsequently, the determination procedure by the determination system 1 will be described. Fig. 7 is a flow chart of the determination using the determination system shown in Fig. 4. Here, the present invention will be described using fluorescence information based on a nucleic acid being measured which is bound to a probe on a microarray and taking an example of the case where averages as "the expression states of genes selected from the gene clusters" are used to perform determination. However, the present invention is not limited only to the embodiment.

In step S1-1, the acquisition unit 301 of the determination system 1 first obtains fluorescence information from the measurement device 2. In step S1-2, the calculation unit 303 calculates fluorescence intensity from the fluorescence information obtained by the acquisition unit 301 and sends it to the memory unit 302.

Subsequently, in step S1-3, the calculation unit 303 calculates an average of the expression levels of a plurality of selected genes based on the fluorescence intensity stored in the memory unit 302.

Thereafter, in step S1-4, the determination unit 304 determines behavior of thyroid tumor using an average calculated by the calculation unit 303 and determination criteria stored in the memory unit 302. The determination results are sent to the output unit 305.

Thereafter, in step S1-5, the output unit 305 outputs the determination results, allows the display unit 3c to display them, and allows the printer to print them.

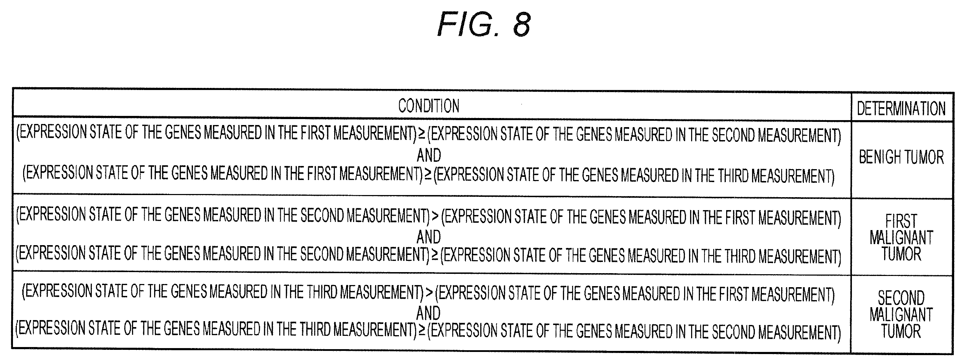

Fig. 8 is a view showing the method for determining behavior of thyroid tumor according to the determination of the present invention. When the conditions shown in the left column of Fig. 8 are satisfied, determination results shown in the right column of Fig. 8 are obtained. In this regard, an inequality sign ">" in Fig.8 means that the expression state shown on the left side is accelerated more than the expression state shown on the right side is.

The inequality sign with an equal sign "≥" shows that the expression state shown on the left side is accelerated more than or equal to the expression state shown on the right side.

Hereinafter, the present invention is more specifically described with reference to Examples; however, the present invention is not limited thereto.

### [Examples]

### Preparation of biological samples

In the following examples, the biological samples obtained as follows were used. First, pathological diagnosis of thyroid lesion tissues extracted from patients with thyroid tumors by surgery was performed. These tissues were classified into one of the cases of adenomatous goiter, follicular adenoma, follicular carcinoma (including poorly-differentiated carcinoma), and papillary carcinoma. These tissues were used as biological samples and cryopreserved at -80°C.

### Example 1

### 1. Measurement of gene expression level with microarray

As the Learning set, tissue specimens of 25 cases which was pathologically classified into follicular neoplasm (11 cases with follicular adenoma and 14 cases with follicular carcinoma) were used. RNAs (100 to 200 ng) extracted from the specimens were amplified with GeneChip (registered trademark) 3' IVT Express Kit (Affymetrix Inc.). The amplified RNAs were added to GeneChip (registered trademark) Human Genome U133 Plus 2.0 Array (Affymetrix Inc.) to react them at 65°C for 16 hours. The RNAs were hybridized to probes on the array. Thereafter, the hybridized probes were stained using GeneChip (registered trademark) Hybridization, Wash, and Stain Kit (Affymetrix Inc.). Subsequently, the luminescence signals were detected using Genechip (registered trademark) Scanner 3000 (Affymetrix Inc.).

### 2. Marker selection

The signal value data (CELL file) obtained by a microarray was normalized by MAS5 using Expression Console (Affymetrix Inc.). Thereafter, the probe whose function was not clear and which had no name was removed. As for probes having overlapping genes, probes other than the probe having the highest signal were removed. As for the signal values of the remaining probes, log-conversion in the base of 2 was performed to calculate CV values of the probes. The top 5000 genes with significant change among the 25 cases (11 cases with follicular adenoma and 14 cases with follicular carcinoma) were extracted. The 5000 genes were Z-scored. On the basis of the results, unsupervised cluster analysis was performed using cluster 3.0 (open source, obtained from the following web site; http://bonsai.hgc.jp/-mdehoon/software/cluster/). Specifically, the analysis procedure is as follows. The Cluster ring tab in the hierarchy of Cluster 3.0 was opened. The box of [Cluster] of [Genes] and the box of [Cluster] of [Arrays] were checked. As for Similarity Metric, the box of [Correlation(Centered)] in [Genes] and [Arrays] was selected. As for Clustering Method, the box of [Centroid linkage] was selected. Thus, the cluster analysis was conducted. The results of the cluster analysis were analyzed using Java Treeview (open source, obtained from the following web site; http://sourceforge.net/projects/jtreeview/). As a result of the cluster analysis using the 5000 genes, it is found that the 25 cases are classified into three groups (Clusters 1, 2, and 3).

Subsequently, in order to narrow down the genes, 37 genes were extracted as genes in which the expression of the genes is increased according to the tree diagram of the gene side. Then, the unsupervised cluster analysis was performed using only the 37 genes thus extracted, and the 25 cases could be classified into three groups. The results are shown in Fig. 1. As shown in the region surrounded by a square in Fig. 1, in Cluster 1 the expressions of 11 genes: AIF1L, CDH16, FAM162B, FGFR2, GJB6, KCNJ13, KIAA1467, SLC25A15, TFCP2L1, TFF3, and TMEM171 are increased, in Cluster 2 the expressions of 17 genes: C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18 are increased, and in Cluster 3 the expression of 9 genes: ANXA1, C13orf33, CYP1B1, FAP, FN1, IL17RD, PDLIM4, RUNX2, and TIMP1 are increased. Hereinafter, the gene clusters in which the expression is increased in Clusters 1, 2, and 3 are referred to as "first gene cluster", "second gene cluster", and "third gene cluster", respectively. Probe IDs of the genes are as shown in Table 1.

Among the 25 cases, 9 cases were classified into Cluster 1, 7 cases were classified into Cluster 2, and 9 cases were classified into Cluster 3. The gene expression-based classification was correlated with the pathology classification. As a result, it was found that follicular adenomas, i.e., benign tumors were concentrated in Cluster 1 (8 cases of 9 cases), and follicular carcinomas, i.e., malignant tumors were concentrated in Clusters 2 and 3 (Cluster 2: 6 cases of 7 cases, Cluster 3: 7 cases of 9 cases). The results are shown in Table 2 (conveniently, the tumor of the case classified into Cluster 2 is referred to as "first malignant tumor", and the tumor of the case classified into Cluster 3 is referred to as "second malignant tumor").

**[Table 2]**

| Cluster analysis | | 37 genes | | |
|---|---|---|---|---|
| | | Cluster 1 | Cluster 2 | Cluster 3 |
| | | Benign tumor | First malignant tumor | Second malignant tumor |
| Pathological classification | Follicular adenoma | 8 | 1 | 2 |
| | Follicular carcinoma | 1 | 6 | 7 |

Accordingly, a total of 37 genes obtained by combining genes in which the expression was increased in Clusters 1, 2 and 3 were used and follicular neoplasms were classified into three groups. The cases classified into Cluster 1 were determined as follicular adenoma, and the cases classified into Cluster 2 or 3 were determined as follicular carcinoma.

### Example 2

Twenty genes below were extracted from the 37 genes and the cluster analysis was performed in the same manner as in Example 1. As a result, 25 cases were classified into three groups in the same manner as in Example 1. In Cluster 1 the expressions of 6 genes: AIF1L, FAM162B, FGFR2, GJB6, KCNJ13, and TFF3 are increased, in Cluster 2 the expressions of 7 genes: FAM125A, MAP2K2, MTG1, PFKL, PTDSS2, SF3A2, and WDR18 are increased, and in Cluster 3 the expressions of 7 genes: ANXA1, CYP1B1, FAP, IL17RD, PDLIM4, RUNX2, and TIMP1 are increased. Among the 25 cases, 11 cases were classified into Cluster 1, 6 cases were classified into Cluster 2, and 8 cases were classified into Cluster 3. The results obtained by correlating the gene expression-based classification with the pathological classification are shown in Table 3.

**[Table 3]**

| Cluster analysis | | 20 genes | | |
|---|---|---|---|---|
| | | Cluster 1 | Cluster 2 | Cluster 3 |
| | | Benign tumor | First malignant tumor | Second malignant tumor |
| Pathological classification | Follicular adenoma | 8 | 1 | 2 |
| | Follicular carcinoma | 3 | 5 | 6 |

As shown in Table 3, it was found that follicular adenomas were concentrated in Cluster 1 (8 cases of 11 cases) and follicular carcinomas were concentrated in Clusters 2 and 3 (Cluster 2: 5 cases of 6 cases, Cluster 3: 6 cases of 8 cases). Accordingly, a total of 20 genes obtained by combining genes in which the expression was increased in Clusters 1, 2 and 3 were used and follicular neoplasms were classified into three groups. The cases classified into Cluster 1 were determined as follicular adenoma, and the cases classified into Cluster 2 or 3 were determined as follicular carcinoma.

### Example 3

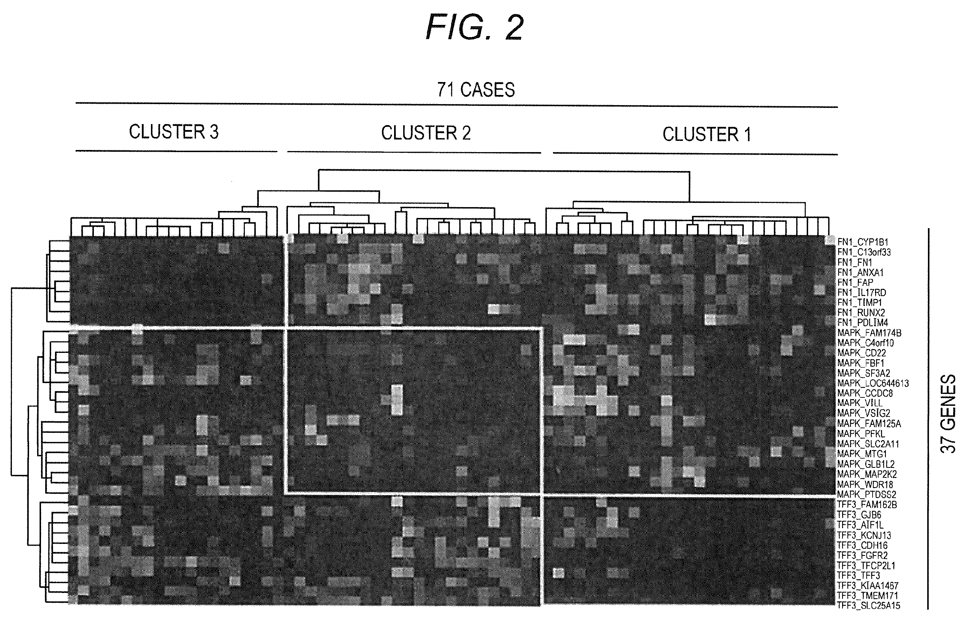

It was examined whether the cluster was reproduced even when a specimen different from the tissue specimen used in Example 1 was used. As the Validation set, tissue specimens in 71 cases different from the Learning set (33 cases with follicular adenoma, 38 cases with follicular carcinoma) were used. RNAs were extracted from the specimens and the expression levels of the 37 genes were measured using the microarray in the same manner as described in Example 1. The signal value data (CELL file) obtained by a microarray was normalized by MAS5 using Expression Console (Affymetrix Inc.). The signal values of the 37 genes selected in Example 1 were extracted. The resulting values were subjected to log-conversion in the base of 2 and further the log-converted values were Z-scored. On the basis of the results, unsupervised cluster analysis was performed using cluster 3.0. The procedure of cluster analysis is the same as in Example 1. As a result, the 71 cases could be classified into three groups (refer to Fig. 2). Among the 71 cases, 27 cases were classified into Cluster 1, 24 cases were classified into Cluster 2, and 20 cases were classified into Cluster 3. The results obtained by correlating the gene expression-based classification with the pathological classification are shown in Table 4.

**[Table 4]**

| Cluster analysis | | 37 genes | | |
|---|---|---|---|---|
| | | Cluster 1 | Cluster 2 | Cluster 3 |
| | | Benign tumor | First malignant tumor | Second malignant tumor |
| Pathological classification | Follicular adenoma | 21 | 6 | 6 |
| | Follicular carcinoma | 6 | 18 | 14 |

As shown in Table 4, it was found that follicular adenomas were concentrated in Cluster 1 (21 cases of 27 cases) and follicular carcinomas were concentrated in Clusters 2 and 3 (Cluster 2: 18 cases of 24 cases, Cluster 3: 14 cases of 20 cases). Thus, as for the determination of behavior of thyroid tumor based on the analysis of the expression levels of the 37 genes, good reproducibility was obtained. Even when the 20 genes were used, the same tendency was observed.

### Example 4

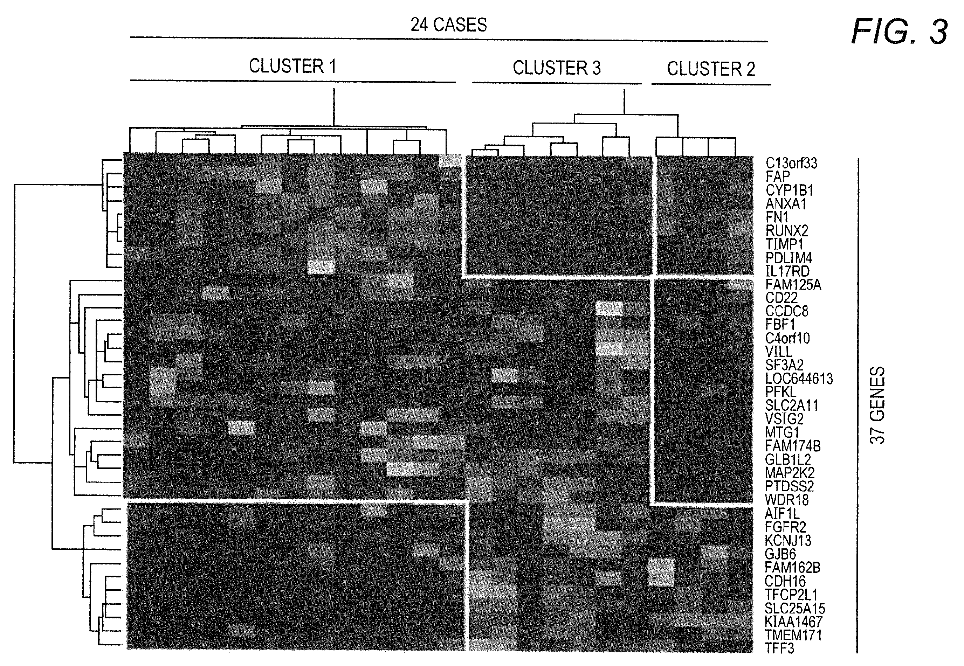

It was examined whether the cluster was reproduced even when adenomatous goiter and papillary carcinoma as specimens were added to follicular adenoma and follicular carcinoma. As for 14 cases with benign tumor (8 cases with adenomatous goiter and 6 cases with follicular adenoma) and 10 cases with malignant tumors (4 cases with papillary carcinoma and 6 cases with follicular carcinoma), RNAs were extracted from the tissue specimens and the expression levels of the 37 genes were measured using the microarray in the same manner as described in Example 1. The procedure of cluster analysis is the same as in Example 1. As a result, the 24 cases could be classified into three groups (refer to Fig. 3). Among the 24 cases, 13 cases were classified into Cluster 1, 4 cases were classified into Cluster 2, and 7 cases were classified into Cluster 3. The results obtained by correlating the gene expression-based classification with the pathological classification are shown in Table 5.

**[Table 5]**

| Cluster analysis | | 37 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 13 | 0 | 1 |
| | Malignant tumor | 0 | 4 | 6 |

As shown in Table 5, it was found that benign tumors were concentrated in Cluster 1 (13 cases of 13 cases) and malignant tumors were concentrated in Clusters 2 and 3 (Cluster 2: 4 cases of 4 cases, Cluster 3: 6 cases of 7 cases). Thus, even if tumor tissues of different types from those of the tumor tissues used when extracting the 37 genes were used as specimens, good reproducibility was obtained in the determination of behavior of thyroid tumor. Accordingly, the 37 genes were used and thyroid tumors were classified into three groups. It was found that the cases classified into Cluster 1 were determined as benign tumor, and the cases classified into Cluster 2 or 3 were determined as malignant tumor.

### Example 5

In this example, in order to perform cluster classification of each case (determination of behavior of tumor) more accurately, it is intended to construct a determination formula for the expression levels of the 37 genes. As biological samples, tissue specimens in 45 cases with benign tumor (adenomatous goiter and follicular adenoma) and 42 cases with malignant tumors (papillary carcinoma and follicular carcinoma) were used. RNAs were extracted from the specimens and the expression levels of the 37 genes (signal values from probes) were measured using the microarray in the same manner as described in Example 1. The signal values were subjected to log-conversion in the base of 2. In this example, averages of the log-converted signal values as for the first, second, and third gene clusters were compared, and the determination formula for classifying each case into each Cluster was tried (hereinafter, also referred to as "determination formula (I)").

Here, the procedure of determination by the determination formula (I) will be described. First, an average of the signal values in each cluster of the first, second, and third gene clusters was calculated. Then, the resulting averages of the first, second, and third gene clusters were compared in the respective cases. The respective cases were classified into the cluster to which the gene cluster having the highest average (that is, the gene cluster in which the expression level is the most increased) belongs.

The determination formula (I) was applied to all the 87 cases and the cluster classification was performed. As a result, among the 87 cases, 38 cases were classified into Cluster 1, 23 cases were classified into Cluster 2, and 26 cases were classified into Cluster 3. The results obtained by correlating the determination formula-based classification with the pathological classification are shown in Table 6.

**[Table 6]**

| Determination formula (I) | | 37 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 32 | 6 | 7 |
| | Malignant tumor | 6 | 17 | 19 |

As shown in Table 6, it was found that benign tumors were concentrated in Cluster 1 (32 cases of 38 cases) and malignant tumors were concentrated in Clusters 2 and 3 (Cluster 2: 17 cases of 23 cases, Cluster 3: 19 cases of 26 cases). As the performance of determination by the determination formula (I), a good performance such that the sensitivity was 86%, the specificity was 71%, the concordance rate was 78%, the PPV was 73%, and the NPV was 84% was obtained. The determination method using the 37 genes showed excellent specificity and PPV as compared with the determination method by the Afirma Thyroid FNA Analysis (a product commercially available from Veracyte, Inc.).

### Example 6

The 20 genes used in Example 2 were used and classifications of 45 cases with benign tumors (adenomatous goiter and follicular adenoma) and 42 cases with malignant tumors (papillary carcinoma and follicular carcinoma) were performed by the determination formula (I) in the same manner as described in Example 5. As a result, among the 87 cases, 43 cases were classified into Cluster 1, 25 cases were classified into Cluster 2, and 19 cases were classified into Cluster 3. The results obtained by correlating the determination formula-based classification with the pathological classification are shown in Table 7.

**[Table 7]**

| Determination formula (I) | | 20 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 34 | 7 | 4 |
| | Malignant tumor | 9 | 18 | 15 |

As shown in Table 7, it was found that benign tumors were concentrated in Cluster 1 (34 cases of 43 cases) and malignant tumors were concentrated in Clusters 2 and 3 (Cluster 2: 18 cases of 25 cases, Cluster 3: 15 cases of 19 cases). As the performance of determination by the determination formula (I), a good performance such that the sensitivity was 79%, the specificity was 76%, the concordance rate was 77%, the PPV was 75%, and the NPV was 79% was obtained. The determination method using the 20 genes showed excellent specificity and PPV as compared with the determination method by the Afirma Thyroid FNA Analysis (a product commercially available from Veracyte, Inc.).

### Example 7

In this example, it is intended to construct a determination formula different from that of Example 5. As biological samples, tissue specimens in 45 cases with benign tumor (adenomatous goiter and follicular adenoma) and 42 cases with malignant tumors (papillary carcinoma and follicular carcinoma) were used. RNAs were extracted from the specimens and the expression levels of the 37 genes (signal values from probes) were measured using the microarray in the same manner as described in Example 1. In this example, the determination formula to classify the genes based on the gene expression patterns of the cases calculated by the primary linear equation and distances from the three clusters classified in Example 1 was tried (hereinafter, the formula is referred to as "determination formula (II)").

Here, the procedure of determination by the determination formula (II) will be described. In the determination formula (II), the signal values of the 37 genes as for the Learning set used in Example 1 were plotted. Differences among the expression levels for the respective clusters were used to classify the genes into three clusters. The three clusters are the same as Clusters 1, 2, and 3 in Example 1. Then, an average of signal values of the clusters was calculated. A Euclid distance between the average of the clusters and data of signal values of the 87 cases was calculated by the primary linear equation. The distances from the clusters in the respective cases were compared. The respective cases were classified into the cluster with the closest distance. As a result, among the 87 cases, 33 cases were classified into Cluster 1, 35 cases were classified into Cluster 2, and 19 cases were classified into Cluster 3. The results obtained by correlating the determination formula-based classification with the pathological classification are shown in Table 8.

**[Table 8]**

| Determination formula (II) | | 37 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 29 | 10 | 6 |
| | Malignant tumor | 4 | 25 | 13 |

As shown in Table 8, it was found that benign tumors were concentrated in Cluster 1 (29 cases of 33 cases) and malignant tumors were concentrated in Clusters 2 and 3 (Cluster 2: 25 cases of 35 cases, Cluster 3: 13 cases of 19 cases). As the performance of determination by the determination formula (II), a good performance such that the sensitivity was 90%, the specificity was 64%, the concordance rate was 77%, the PPV was 70%, and the NPV was 88% was obtained. The determination method using the 37 genes showed excellent specificity and PPV as compared with the determination method by the Afirma Thyroid FNA Analysis (a product commercially available from Veracyte, Inc.).

### Example 8

The 20 genes used in Example 2 were used and classifications of 45 cases with benign tumors (adenomatous goiter and follicular adenoma) and 42 cases with malignant tumors (papillary carcinoma and follicular carcinoma) were performed by the determination formula (II) in the same manner as described in Example 7. As a result, among the 87 cases, 33 cases were classified into Cluster 1, 29 cases were classified into Cluster 2, and 25 cases were classified into Cluster 3. The results obtained by correlating the determination formula-based classification with the pathological classification are shown in Table 9.

**[Table 9]**

| Determination formula (II) | | 20 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 27 | 11 | 7 |
| | Malignant tumor | 6 | 18 | 18 |

As shown in Table 9, it was found that benign tumors were concentrated in Cluster 1 (27 cases of 33 cases) and malignant tumors were concentrated in Clusters 2 and 3 (Cluster 2: 18 cases of 29 cases, Cluster 3: 18 cases of 25 cases). As the performance of determination by the determination formula (II), a good performance such that the sensitivity was 86%, the specificity was 60%, the concordance rate was 72%, the PPV was 67%, and the NPV was 82% was obtained. The determination method using the 20 genes showed excellent specificity and PPV as compared with the determination method by the Afirma Thyroid FNA Analysis (a product commercially available from Veracyte, Inc.).

### Example 9

In this example, a difference between Cluster 2 and Cluster 3 was examined from the viewpoint of the prognosis in malignant tumor. Among follicular carcinoma, poorly-differentiated carcinoma, widely invasive -type follicular carcinoma, and follicular carcinoma withvascular invasion are generally known as poor-prognostic malignant tumors. Here, the cases used in Example 7 include 15 cases which are malignant tumors with poor prognosis of the cases. Then, it was examined into which Cluster these cases were classified in Example 7. The results are shown in Table 10.

**[Table 10]**

| Determination formula (II) | | 37 genes | | |
|---|---|---|---|---|
| | | Cluster 1 | Cluster 2 | Cluster 3 |
| | | Benign tumor | First malignant tumor | Second malignant tumor |
| Pathological classification | Benign tumor | 0 | 0 | 0 |
| | Malignant tumor | 0 | 11 | 4 |

As shown in Table 10, the number of cases classified into Cluster 1 was 0, the number of cases classified into Cluster 2 was 11, and the number of cases classified into Cluster 3 was 4. Thus, a greater number of cases with poor prognosis were concentrated in Cluster 2. This suggested that the case classified into Cluster 2 (the first malignant tumor) had a higher possibility of being a malignant tumor with poor prognosis as compared with the case classified into Cluster 3 (the second malignant tumor).

## Claims

1. A method for determining behavior of thyroid tumor of a subject, comprising:
a first measurement step comprising measuring expression levels of at least 6 genes selected from the group consisting of AIF1L, CDH16, FAM162B, FGFR2, GJB6, KCNJ13, KIAA1467, SLC25A15, TFCP2L1, TFF3, and TMEM171 in a sample collected from thyroid lesion tissues of the subject;
a second measurement step comprising measuring expression levels of at least 7 genes selected from the group consisting of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18 in the sample;
a third measurement step comprising measuring expression levels of at least 7 genes selected from the group consisting of ANXA1, C13orf33, CYP1B1, FAP, FN1, IL17RD, PDLIM4, RUNX2, and TIMP1 in the sample; and
determination step comprising determining whether a thyroid tumor in a subject is benign or malignant based on the results of first, second, and third measurements, wherein in the determination step,
when the expression state of the genes measured in the first measurement is more than or equal to the expression state of the genes measured in the second measurement, and when the expression state of the genes measured in the first measurement is more than or equal to the expression state of the genes measured in the third measurement, the thyroid tumor of the subject is determined to be benign,
when the expression state of the genes measured in the second measurement is more than the genes measured in the first measurement, and when the expression state of the genes measured in the second measurement is more than or equal to the expression state of the genes measured in the third measurement, the thyroid tumor of the subject is determined to a first malignant tumor, and
when the expression state of the genes measured in the third measurement is more than the expression states of the genes measured in the first and second measurements, the thyroid tumor of the subject is determined to a second malignant tumor, wherein the first malignant tumor is a malignant tumor with poor prognosis, and
the second malignant tumor is a malignant tumor with good prognosis.

2. The method according to claim 1, wherein
the benign tumor is at least one of follicular adenoma, adenomatous nodules, and cysts,
the first malignant tumor is at least one of undifferentiated carcinoma, poorly-differentiated carcinoma, and follicular carcinoma with widely invasive and/or vascular invasion, and
the second malignant tumor is at least one of papillary carcinoma and follicular carcinoma without widely invasive and vascular invasion.

3. The method according to any of claims 1 to 2, wherein
in the first measurement, the expression levels of at least 6 genes: AIF1L, FAM162B, FGFR2, GJB6, KIAA1467, and TFF3 are measured,
in the second measurement, the expression levels of at least 7 genes: FAM174B, MAP2K2, MTG1, PFKL, PTDSS2, SF3A2, and WDR18 are measured, and in the third measurement, the expression levels of at least 7 genes: ANXA1, CYP1B1, FAP, IL17RD, PDLIM4, RUNX2, and TIMP1 are measured.

4. The method according to any of claims 1 to 3, wherein
a microarray or a nucleic acid amplification method is used to measure the gene expression levels in the first, second, and third measurements.

5. The method according to any of claims 1 to 4, wherein
the sample contains cells derived from the thyroid of the subject.

6. The method according to any of claims 1 to 5, further comprising
first calculation step comprising calculating a central tendency of expression levels of genes measured in the first measurement,
second calculation step comprising calculating a central tendency of expression levels of genes measured in the second measurement, and
third calculation step comprising calculating a central tendency of expression levels of genes measured in the third measurement.

7. The method according to claim 6, wherein
the central tendency is an average, a median, a maximum, or a mode.

8. A system adapted to a method for determining behavior of thyroid tumor, comprising:
a processor, and
a memory, under control of said processor, including software instructions adapted to enable the system to perform operations comprising:
determining whether a thyroid tumor in a subject is benign or malignant based on the results of first, second, and third measurements;
wherein the expression levels of at least 6 genes selected from the group consisting of AIF1L, CDH16, FAM162B, FGFR2, GJB6, KCNJ13, KIAA1467, SLC25A15, TFCP2L1, TFF3, and TMEM171 in a sample from thyroid lesion tissues collected from the subject are measured in the first measurement,
the expression levels of at least 7 genes selected from the group consisting of C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2, and WDR18 in the sample are measured in the second measurement, and
the expression levels of at least 7 genes selected from the group consisting of ANXA1, C13orf33, CYP1B1, FAP, FN1, IL17RD, PDLIM4, RUNX2, and TIMP1 in the sample are measured in the third measurement, wherein
in the operations,
when the expression state of the genes measured in the first measurement is more than or equal to the expression state of the genes measured in the second measurement, and when the expression state of the genes measured in the first measurement is more than or equal to the expression state of the genes measured in the third measurement, the thyroid tumor of the subject is determined to be benign,
when the expression state of the genes measured in the second measurement is more than the genes measured in the first measurement, and when the expression state of the genes measured in the second measurement is more than or equal to the expression state of the genes measured in the third measurement, the thyroid tumor of the subject is determined to a first malignant tumor, and
when the expression state of the genes measured in the third measurement is more than the expression states of the genes measured in the first and second measurements, the thyroid tumor of the subject is determined to a second malignant tumor, wherein
the first malignant tumor is a malignant tumor with poor prognosis, and
the second malignant tumor is a malignant tumor with good prognosis.

## Patentansprüche

1. Verfahren zur Bestimmung des Verhaltens eines Schilddrüsen-Tumors einer Person, umfassend:
einen ersten Messschritt, umfassend Messung von Expressionsniveaus von mindestens 6 Genen, ausgewählt aus der Gruppe, bestehend aus AIF1L, CDH16, FAM162B, FGFR2, GJB6, KCNJ13, KIAA1467, SLC25A15, TFCP2L1, TFF3 und TMEM171, in einer Probe aus Schilddrüsen-Läsionsgewebe der Person;
einen zweiten Messschritt, umfassend Messung von Expressionsniveaus von wenigstens 7 Genen, ausgewählt aus der Gruppe, bestehend aus C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2 und WDR18, in der Probe;
einen dritten Messschritt, umfassend Messung von Expressionsniveaus von wenigstens 7 Genen, ausgewählt aus der Gruppe, bestehend aus ANXA1, C13orf33, CYP1B1, FAP, FN1, IL17RD, PDLIM4, RUNX2 und RIMP1, in der Probe; und
Bestimmungsschritt, umfassend Bestimmung, ob ein Schilddrüsen-Tumor bei einer Person gutartig oder bösartig ist, basierend auf den Ergebnissen aus ersten, zweiten und dritten Messungen, wobei in dem Bestimmungsschritt,
wenn der bei der ersten Messung gemessene Expressionszustand der Gene größer oder gleich ist zu dem bei der zweiten Messung gemessenen Expressionszustand der Gene und wenn der bei der ersten Messung gemessene Expressionszustand der Gene größer oder gleich ist zu dem bei der dritten Messung gemessenen Expressionszustand der Gene, der Schilddrüsen-Tumor von der Person als gutartig bestimmt wird,
wenn der bei der zweiten Messung gemessene Expressionszustand der Gene größer ist als der bei der ersten Messung gemessene Expressionszustand der Gene und wenn der bei der zweiten Messung gemessene Expressionszustand der Gene größer oder gleich ist zu dem bei der dritten Messung gemessenen Expressionszustand der Gene, der Schilddrüsen-Tumor von der Person als ein erster bösartiger Tumor bestimmt wird, und
wenn der bei der dritten Messung gemessene Expressionszustand der Gene größer ist als die bei den ersten und zweiten Messungen gemessenen Expressionszustände der Gene, der Schilddrüsen-Tumor der Person als ein zweiter bösartiger Tumor bestimmt wird, wobei
der erste bösartige Tumor ein bösartiger Tumor mit schlechter Prognose und der zweite bösartige Tumor ein bösartiger Tumor mit guter Prognose ist.

2. Verfahren gemäß Anspruch 1, wobei
der gutartige Tumor wenigstens einer aus follikulärem Adenom, adenomatösen Knötchen und Zysten ist,
der erste bösartige Tumor wenigstens einer aus undifferenziertem Karzinom, schlecht differenziertem Karzinom und follikulärem Karzinom mit breit invasiver und/oder vaskulärer Invasion ist und
der zweite bösartige Tumor wenigstens einer aus papillarem Karzinom und follikulärem Karzinom ohne breit invasiver und vaskulärer Invasion ist.

3. Verfahren gemäß einem der Ansprüche 1 bis 2, wobei
bei der ersten Messung die Expressionsniveaus von wenigstens 6 Genen: AIF1L, FAM162B, FGFR2, GJB6, KIAA1467 und TFF3, gemessen werden,
bei der zweiten Messung die Expressionsniveaus von wenigstens 7 Genen: FAM174B, MAP2K2, MTG1, PFKL, PTDSS2, SF3A2 und WDR18, gemessen werden und
bei der dritten Messung die Expressionsniveaus von wenigstens 7 Genen: ANXA1, CYP1B1, FAP, IL17RD, PDLIM3, RUNX2 und TIMP1, gemessen werden.

4. Verfahren gemäß einem der Ansprüche 1 bis 3, wobei
ein Microarray oder ein Nukleinsäure-Amplifikationsverfahren zur Messung der Genexpressionsniveaus bei den ersten, zweiten und dritten Messungen verwendet wird.

5. Verfahren gemäß einem der Ansprüche 1 bis 4, wobei
die Probe Zellen enthält, welche von der Schilddrüse der Person stammen.

6. Verfahren gemäß einem der Ansprüche 1 bis 5, ferner umfassend
ersten Berechnungsschritt, umfassend Berechnung einer zentralen Tendenz der Bei der ersten Messung gemessenen Expressionsniveaus von Genen,
zweiten Berechnungsschritt, umfassend Berechnung einer zentralen Tendenz der Bei der zweiten Messung gemessenen Expressionsniveaus von Genen und
dritten Berechnungsschritt, umfassend Berechnung einer zentralen Tendenz der Bei der dritten Messung gemessenen Expressionsniveaus von Genen.

7. Verfahren gemäß Anspruch 6, wobei
die zentrale Tendenz ein Mittelwert, ein Median, ein Maximum oder ein Modus ist.

8. System, angepasst an ein Verfahren zur Bestimmung des Verhaltens eines Schilddrüsen-Tumors, umfassend:
einen Prozessor und
einen Speicher unter der Kontrolle des Prozessors, einschließlich Software-Anweisungen, angepasst, um das System in die Lage zu versetzen, Arbeitsschritte durchzuführen, umfassend:
Bestimmung, ob ein Schilddrüsen-Tumor bei einer Person gutartig oder bösartig ist, basierend auf den Ergebnissen aus ersten, zweiten und dritten Messungen;
wobei die Expressionsniveaus von wenigstens 6 Genen, ausgewählt aus der Gruppe, bestehend aus AIF1L, CDH16, FAM162B, FGFR2, GJB6, KCNJ13, KIAA1467, SLC25A15, TFCP2L1, TFF3 und TMEM171, in einer Probe aus Schilddrüsen-Läsionsgewebe der Person bei der ersten Messung gemessen werden,
die Expressionsniveaus von wenigstens 7 Genen, ausgewählt aus der Gruppe, bestehend aus C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2 und WDR18, in der Probe bei der zweiten Messung gemessen werden und
die Expressionsniveaus von wenigstens 7 Genen, ausgewählt aus der Gruppe, bestehend aus ANXA1, C13orf33, CYP1B1, FAP, FN1, IL17RD, PDLIM4, RUNX2 und TIMP1, in der Probe bei der dritten Messung gemessen werden, wobei
bei den Arbeitsschritten,
wenn der bei der ersten Messung gemessene Expressionszustand der Gene größer oder gleich ist zu dem bei der zweiten Messung gemessenen Expressionszustand der Gene und wenn der bei der ersten Messung gemessene Expressionszustand der Gene größer oder gleich ist zu dem bei der dritten Messung gemessenen Expressionszustand der Gene, der Schilddrüsen-Tumor von der Person als gutartig bestimmt wird,
wenn der bei der zweiten Messung gemessene Expressionszustand der Gene größer ist als der bei der ersten Messung gemessene Expressionszustand der Gene und wenn der bei der zweiten Messung gemessene Expressionszustand der Gene größer oder gleich ist dem bei der dritten Messung gemessenen Expressionszustand der Gene, der Schilddrüsen-Tumor von der Person als ein erster bösartiger Tumor bestimmt wird, und
wenn der bei der dritten Messung gemessene Expressionszustand der Gene größer ist als der bei den ersten und zweiten Messungen gemessene Expressionszustand der Gene, der Schilddrüsen-Tumor der Person als ein zweiter bösartiger Tumor bestimmt wird, wobei
der erste bösartige Tumor ein bösartiger Tumor mit schlechter Prognose und der zweite bösartige Tumor ein bösartiger Tumor mit guter Prognose ist.

## Revendications

1. Procédé permettant de déterminer le comportement d'une tumeur thyroïdienne d'un sujet, comprenant :
une première étape de mesure comprenant le fait de mesurer des niveaux d'expression d'au moins 6 gènes sélectionnés dans le groupe constitué de AIF1L, CDH16, FAM162B, FGFR2, GJB6, KCNJ13, KIAA1467, SLC25A15, TFCP2L1, TFF3 et TMEM171 dans un échantillon prélevé de tissus de lésions thyroïdiennes du sujet ;
une deuxième étape de mesure comprenant le fait de mesurer des niveaux d'expression d'au moins 7 gènes sélectionnés dans le groupe constitué de C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2 et WDR18 dans l'échantillon ;
une troisième étape de mesure comprenant le fait de mesurer des niveaux d'expression d'au moins 7 gènes sélectionnés dans le groupe constitué de ANXA1, C13orf33, CYP1B1, FAP, FN1, IL17RD, PDLIM4, RUNX2 et TIMP1 dans l'échantillon ; et
une étape de détermination comprenant le fait de déterminer si une tumeur thyroïdienne chez un sujet est bénigne ou maligne sur la base des résultats des première, deuxième et troisième mesures, où dans l'étape de détermination,
lorsque l'état d'expression des gènes mesurés dans la première mesure est supérieur ou égal à l'état d'expression des gènes mesurés dans la deuxième mesure, et lorsque l'état d'expression des gènes mesurés dans la première mesure est supérieur ou égal à l'état d'expression des gènes mesurés dans la troisième mesure, la tumeur thyroïdienne du sujet est déterminée comme étant bénigne,
lorsque l'état d'expression des gènes mesurés dans la deuxième mesure est supérieur à celui des gènes mesurés dans la première mesure, et lorsque l'état d'expression des gènes mesurés dans la deuxième mesure est supérieur ou égal à l'état d'expression des gènes mesurés dans la troisième mesure, la tumeur thyroïdienne du sujet est déterminée comme étant une première tumeur maligne, et
lorsque l'état d'expression des gènes mesurés dans la troisième mesure est supérieur aux états d'expression des gènes mesurés dans les première et deuxième mesures, la tumeur thyroïdienne du sujet est déterminée comme étant une deuxième tumeur maligne, où
la première tumeur maligne est une tumeur maligne avec un mauvais pronostic, et
la deuxième tumeur maligne est une tumeur maligne avec un bon pronostic.

2. Procédé selon la revendication 1, dans lequel
la tumeur bénigne est au moins l'un parmi l'adénome folliculaire, des nodules adénomateux, et des kystes,
la première tumeur maligne est au moins l'un parmi un carcinome indifférencié, un carcinome peu différencié, et un carcinome folliculaire largement invasif et/ou à invasion vasculaire, et
la deuxième tumeur maligne est au moins l'un parmi un carcinome papillaire et un carcinome folliculaire qui n'est pas largement invasif et sans invasion vasculaire.

3. Procédé selon l'une des revendications 1 et 2, dans lequel
dans la première mesure, les niveaux d'expression d'au moins 6 gènes : AIF1L, FAM162B, FGFR2, GJB6, KIAA1467 et TFF3 sont mesurés,
dans la deuxième mesure, les niveaux d'expression d'au moins 7 gènes : FAM174B, MAP2K2, MTG1, PFKL, PTDSS2, SF3A2 et WDR18 sont mesurés, et
dans la troisième mesure, les niveaux d'expression d'au moins 7 gènes : ANXA1, CYP1B1, FAP, IL17RD, PDLIM4, RUNX2 et TIMP1 sont mesurés.

4. Procédé selon l'une des revendications 1 à 3, dans lequel
un microréseau ou un procédé d'amplification d'acide nucléique est utilisé pour mesurer les niveaux d'expression des gènes dans les première, deuxième et troisième mesures.

5. Procédé selon l'une des revendications 1 à 4, dans lequel
l'échantillon contient des cellules provenant de la thyroïde du sujet.

6. Procédé selon l'une des revendications 1 à 5, comprenant en outre
une première étape de calcul comprenant le fait de calculer une tendance centrale des niveaux d'expression de gènes mesurés dans la première mesure,
une deuxième étape de calcul comprenant le fait de calculer une tendance centrale des niveaux d'expression de gènes mesurés dans la deuxième mesure, et
une troisième étape de calcul comprenant le fait de calculer une tendance centrale des niveaux d'expression de gènes mesurés dans la troisième mesure.

7. Procédé selon la revendication 6, dans lequel
la tendance centrale est une moyenne, une médiane, un maximum, ou un mode.

8. Système adapté à un procédé permettant de déterminer le comportement d'une tumeur thyroïdienne, comprenant :
un processeur et
une mémoire, sous la commande dudit processeur, comportant des instructions logicielles adaptées pour permettre au système d'effectuer des opérations comprenant le fait :
de déterminer si une tumeur thyroïdienne chez un sujet est bénigne ou maligne sur la base des résultats des première, deuxième et troisième mesures ;
où les niveaux d'expression d'au moins 6 gènes sélectionnés dans le groupe constitué de AIF1L, CDH16, FAM162B, FGFR2, GJB6, KCNJ13, KIAA1467, SLC25A15, TFCP2L1, TFF3 et TMEM171 dans un échantillon provenant de tissus de lésions thyroïdiennes prélevés du sujet sont mesurés dans la première mesure,
les niveaux d'expression d'au moins 7 gènes sélectionnés dans le groupe constitué de C4orf10, CCDC8, CD22, FAM125A, FAM174B, FBF1, GLB1L2, LOC644613, MAP2K2, MTG1, PFKL, PTDSS2, SF3A2, SLC2A11, VILL, VSIG2 et WDR18 dans l'échantillon sont mesurés dans la deuxième mesure, et
les niveaux d'expression d'au moins 7 gènes sélectionnés dans le groupe constitué de ANXA1, C13orf33, CYP1B1, FAP, FN1, IL17RD, PDLIM4, RUNX2 et TIMP1 dans l'échantillon sont mesurés dans la troisième mesure, où
dans les opérations,
lorsque l'état d'expression des gènes mesurés dans la première mesure est supérieur ou égal à l'état d'expression des gènes mesurés dans la deuxième mesure, et lorsque l'état d'expression des gènes mesurés dans la première mesure est supérieur ou égal à l'état d'expression des gènes mesurés dans la troisième mesure, la tumeur thyroïdienne du sujet est déterminée comme étant bénigne,
lorsque l'état d'expression des gènes mesurés dans la deuxième mesure est supérieur à celui des gènes mesurés dans la première mesure, et lorsque l'état d'expression des gènes mesurés dans la deuxième mesure est supérieur ou égal à l'état d'expression des gènes mesurés dans la troisième mesure, la tumeur thyroïdienne du sujet est déterminée comme étant une première tumeur maligne, et
lorsque l'état d'expression des gènes mesurés dans la troisième mesure est supérieur aux états d'expression des gènes mesurés dans les première et deuxième mesures, la tumeur thyroïdienne du sujet est déterminée comme étant une deuxième tumeur maligne, où
la première tumeur maligne est une tumeur maligne avec un mauvais pronostic, et
la deuxième tumeur maligne est une tumeur maligne avec un bon pronostic.
